(19) **Europäisches Patentamt / European Patent Office / Office européen des brevets**

(11) **EP 3 897 605 B1**

(12) **EUROPEAN PATENT SPECIFICATION**

(45) Date of publication and mention of the grant of the patent:
**22.11.2023 Bulletin 2023/47**

(21) Application number: **20838273.9**

(22) Date of filing: **23.12.2020**

(51) International Patent Classification (IPC):
**A61K 31/196** (2006.01)  **A61K 31/4184** (2006.01)
**A61K 31/4439** (2006.01)  **A61K 31/454** (2006.01)
**A61K 31/502** (2006.01)  **A61K 31/5025** (2006.01)
**A61K 31/513** (2006.01)  **A61K 31/519** (2006.01)
**A61K 31/55** (2006.01)  **A61K 31/58** (2006.01)
**A61P 35/00** (2006.01)

(52) Cooperative Patent Classification (CPC):
(C-Sets available)
**A61K 31/196; A61K 31/4184; A61K 31/4439; A61K 31/454; A61K 31/502; A61K 31/5025; A61K 31/513; A61K 31/519; A61K 31/55; A61K 31/58; A61P 35/00**   (Cont.)

(86) International application number:
**PCT/GB2020/053365**

(87) International publication number:
**WO 2021/130498 (01.07.2021 Gazette 2021/26)**

(54) **COMBINATION THERAPY**

KOMBINATIONSTHERAPIE

POLYTHÉRAPIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2019 GB 201919301**

(43) Date of publication of application:
**27.10.2021 Bulletin 2021/43**

(73) Proprietor: **Turbine Simulated Cell Technologies Ltd**
**London EC2Y 5EB (GB)**

(72) Inventors:
• **BARTHA, Árpád László**
**London Wall London EC2Y 5EB (GB)**
• **ELEK, Boldizsar Frigyes**
**London Wall London EC2Y 5EB (GB)**
• **FEKETE, Iván**
**London Wall London EC2Y 5EB (GB)**
• **KAMP, Sebestyén**
**London Wall London EC2Y 5EB (GB)**
• **KISISTÓK, Judit**
**London Wall London EC2Y 5EB (GB)**
• **PAPP, Orsolya**
**London Wall London EC2Y 5EB (GB)**
• **VERES, Daniel**
**London Wall London EC2Y 5EB (GB)**

(74) Representative: **Stratagem IPM Limited**
**Meridian Court**
**Comberton Road**
**Toft, Cambridge CB23 2RY (GB)**

(56) References cited:
**WO-A1-2008/124838  WO-A1-2018/067575**
**WO-A1-2018/191153  US-B1- 9 132 120**

• **CZYZ MALGORZATA ET AL: "PARP1 inhibitor olaparib (Lynparza) exerts synthetic lethal effect against ligase 4-deficient melanomas", ONCOTARGET, vol. 7, no. 46, 15 November 2016 (2016-11-15), pages 75551-75560, XP055787729, DOI: 10.18632/oncotarget.12270**

- **SAMUEL F. BUNTING ET AL: "53BP1 Inhibits Homologous Recombination in Brca1-Deficient Cells by Blocking Resection of DNA Breaks", CELL, vol. 141, no. 2, 1 April 2010 (2010-04-01) , pages 243-254, XP055430025, Amsterdam NL ISSN: 0092-8674, DOI: 10.1016/j.cell.2010.03.012**
- **Tobin Lisa A. ET AL: "Targeting Abnormal DNA Repair in Therapy-Resistant Breast Cancers", MOLECULAR CANCER RESEARCH, vol. 10, no. 1, 23 November 2011 (2011-11-23), pages 96-107, XP055787533, US ISSN: 1541-7786, DOI: 10.1158/1541-7786.MCR-11-0255**
- **CHEN XI ET AL: "Rational design of human DNA ligase inhibitors that target cellular DNA replication and repair", CANCER RESEARCH, AMERICAN ASSOCIATION FOR CANCER RESEARCH, US, vol. 68, no. 9, 1 May 2008 (2008-05-01), pages 3169-3177, XP002551053, ISSN: 0008-5472, DOI: 10.1158/0008-5472.CAN-07-6636**

(52) Cooperative Patent Classification (CPC): (Cont.)

C-Sets
**A61K 31/196, A61K 2300/00;
A61K 31/4184, A61K 2300/00;
A61K 31/4439, A61K 2300/00;
A61K 31/454, A61K 2300/00;
A61K 31/502, A61K 2300/00;
A61K 31/5025, A61K 2300/00;
A61K 31/513, A61K 2300/00;
A61K 31/519, A61K 2300/00;
A61K 31/55, A61K 2300/00;
A61K 31/58, A61K 2300/00**

**Description**

This application relates to the combination of a DNA ligase inhibitor and a PARP inhibitor in synergistically effective amounts. In particular, though not exclusively, the pharmaceutical combinations described herein may be useful in treating cancers, drug resistance and/or drug resistant cancers. This application also relates to a method for selecting a combination of a DNA ligase inhibitor and a PARP inhibitor.

**BACKGROUND OF THE INVENTION**

Treating cancer and cancers that show drug resistance is a significant problem leading to the suffering and deaths of millions worldwide.
For example, it is estimated that over a million new cases of cancer will be diagnosed each year in the United States alone, and over half a million people in the US will die from the disease each year.
The most common cancers include breast cancer, lung and bronchus cancer, prostate cancer, colon and rectum cancer, melanoma of the skin, bladder cancer, non-Hodgkin lymphoma, kidney and renal pelvis cancer, endometrial cancer, leukaemia, pancreatic cancer, thyroid cancer and liver cancer.
While various treatments are available, there remains a need in the art for improved solutions to the problem of treating cancer and drug resistant cancer.

**SUMMARY OF THE INVENTION**

In a **first aspect** of the invention, there is provided a pharmaceutical combination for use in the treatment of cancer comprising a DNA ligase IV inhibitor and a PARP inhibitor in synergistically effective amounts for simultaneous, separate or sequential administration to a subject in need thereof. The scope of the invention is defined by the claims. The references to methods of treatment in the summary and detailed description of the invention in this description are to be interpreted as references to the compounds, pharmaceutical compositions and medicaments of the present invention for use in a method for treatment of the human (or animal) body by therapy.
In an embodiment, the DNA ligase is a DNA ligase IV inhibitor. In an embodiment, the DNA ligase inhibitor has an inhibitory effect towards DNA ligase I, DNA ligase III and/or DNA ligase IV. In an embodiment, the DNA ligase inhibitor has an inhibitory effect towards DNA ligase I and/or DNA ligase IV. In an embodiment, the DNA ligase inhibitor has an inhibitory effect towards DNA ligase IV. In an embodiment, the DNA ligase inhibitor has a selective inhibitory effect towards DNA ligase IV.
This application relates to the combination of a DNA ligase inhibitor and a PARP inhibitor as specified in the claims in synergistically effective amounts. In particular, though not exclusively, the pharmaceutical combinations described herein may be useful in treating cancers, drug resistance and/or drug resistant cancers. This application also relates to a method for selecting a combination of a DNA ligase inhibitor and a PARP inhibitor.

**BACKGROUND OF THE INVENTION**

Treating cancer and cancers that show drug resistance is a significant problem leading to the suffering and deaths of millions worldwide.
For example, it is estimated that over a million new cases of cancer will be diagnosed each year in the United States alone, and over half a million people in the US will die from the disease each year.
The most common cancers include breast cancer, lung and bronchus cancer, prostate cancer, colon and rectum cancer, melanoma of the skin, bladder cancer, non-Hodgkin lymphoma, kidney and renal pelvis cancer, endometrial cancer, leukaemia, pancreatic cancer, thyroid cancer and liver cancer.
Prior disclosures include: WO2008124838 which discloses methods for treating cancer using compounds that inhibit human DNA ligases; WO2018067575 which relates to the modelling, treatment, reduction of resistence to the treatment, prevention, and/or diagnosis of diseases characterized by the formation of cancers; US9132120 which discloses a method of treating a breast cancer tumor which is non-responsive or intrinsically resistant to anti-estrogen therapy and a method of treating pancreatic cancer which is non-responsive to chemotherapy and/or radiation; and Czyz Malgorzata et al, Oncotarget. 2016; 7:75551-75560, which discloses that melanoma may be "addicted" to double strand break (DSB) repair and targeting this process could sensitize them to the lethal effect of DNA damage.
While various treatments are available, there remains a need in the art for improved solutions to the problem of treating cancer and drug resistant cancer.

## SUMMARY OF THE INVENTION

Herein disclosed is a pharmaceutical combination for use in the treatment of cancer comprising a DNA ligase IV inhibitor and a PARP inhibitor in synergistically effective amounts for simultaneous, separate or sequential administration to a subject in need thereof.

In a first aspect of the invention, there is provided a pharmaceutical combination for use in the treatment of cancer comprising a DNA ligase IV inhibitor and a PARP inhibitor in synergistically effective amounts for simultaneous, separate or sequential administration to a subject in need thereof, wherein the cancer is a drug resistant cancer, wherein the DNA ligase IV inhibitor comprises L189 or SCR7 and the PARP inhibitor comprises Talazoparib or Olaparib.

In an embodiment, the DNA ligase is a DNA ligase IV inhibitor. In an embodiment, the DNA ligase inhibitor has an inhibitory effect towards DNA ligase I, DNA ligase III and/or DNA ligase IV. In an embodiment, the DNA ligase inhibitor has an inhibitory effect towards DNA ligase I and/or DNA ligase IV. In an embodiment, the DNA ligase inhibitor has an inhibitory effect towards DNA ligase IV. In an embodiment, the DNA ligase inhibitor has a selective inhibitory effect towards DNA ligase IV.

The invention makes use of a combination of two inhibitor components in order to impair effective DNA repair, and so to target cancer cells. Each inhibitor acting synergistically in order to prevent cancer cells from proliferating, and/or killing the cancer cells. The combination of the invention has been found to be more effective than the predicted sum of each component additively. This synergistic interaction makes the combination more effective against cancers. This synergy can lead to greater activity, and/or to lower doses of the active components. Beneficially, lower doses of the active components can reduce side effects and can save on costs. Additionally, it is believed that drug resistant cancers may be more effectively treated, in particular cancers resistant to PARP inhibition as monotherapy. The combinations of inhibitors were intelligently identified by computer modelling.

DNA ligase is a type of enzyme that facilitates the joining of DNA strands together by catalysing the formation of a phosphodiester bond (see Pascal JM, O'Brien PJ, Tomkinson AE, Ellenberger T (November 2004); "Human DNA ligase I completely encircles and partially unwinds nicked DNA"; Nature. 432 (7016): 473-8). DNA ligase plays a role in repairing single-strand breaks in duplex DNA in living organisms, but some forms (such as DNA ligase IV) may specifically repair double-strand breaks (i.e. a break in both complementary strands of DNA). Single-strand breaks are repaired by DNA ligase using the complementary strand of the double helix as a template with DNA ligase creating the final phosphodiester bond to fully repair the DNA.

Poly [ADP-ribose] polymerase 1 (PARP-1) is an enzyme that in humans is encoded by the PARP-1 gene (Ha HC, Snyder SH (August 2000); "Poly (ADP-ribose) polymerase-1 in the nervous system"; Neurobiology of Disease. 7 (4): 225-39). PARP-1 is believed to be involved in the repair of ssDNA through base excision repair (BER) mechanisms. PARP-1 inhibition is believed to result in the accumulation of ssDNA lesions, which stall replication forks and ultimately lead to the accumulation of DNA double strand breaks (DSBs). For cell viability, repair of these DSBs is important, and may be reliant on processes such as non-homologous end joining (NHEJ), an alternative form of NHEJ - alternative end-joining (AEJ), and homologous recombination repair (HRR). PARP-1 may also participate in AEJ which functions as a backup to the NHEJ process. PARP-1 inhibition, for example, by the invention could also be used to exploit Ligase IV (LIG4)-deprived cancers.

It is believed that a combination of the deficiencies in the expression of two or more genes may lead to cell death. The deficiencies may be mutations, epigenetic alterations, and/or the inhibition of gene products. Cancer cells, which due to the mutations they possess, may therefore be sensitised to the inhibition of certain gene products over healthy tissue. The invention makes use of this potential sensitivity.

In an embodiment the DNA ligase inhibitor is a strong DNA ligase inhibitor. In an embodiment the activity (e.g. $IC_{50}$) of the DNA ligase is under 50 $\mu$M, under 20 $\mu$M, under 10 $\mu$M, under 5 $\mu$M or under 1 $\mu$M. In an embodiment the activity of the DNA ligase is selective for DNA ligase I and/or DNA ligase IV.

In an embodiment the PARP inhibitor is a strong PARP inhibitor. In an embodiment the activity of the PARP inhibitor is under 50 $\mu$M, under 20 $\mu$M, under 10 $\mu$M, under 5 $\mu$M or under 1 $\mu$M. In an embodiment the activity of the PARP inhibitor is selective for PARP-1.

In an embodiment, the PARP inhibitor is a non-trapping PARP inhibitor. In an embodiment, the PARP inhibitor is a PARP-1 inhibitor which potentiates the process of 'PARP trapping' at a higher concentration than its cellular PARP $IC_{50}$. For example, 'PARP trapping' refers to the small-molecule induced trapping of PARP enzymes at sites of DNA damage and the PARP trapping activity of a compound is not correlated to the catalytic inhibitory properties of said compound. Trapped PARP-DNA complexes are believed to be more cytotoxic than PARP inactivation, and are reliant on processes such as Homologous Recombination R for resolution.

In an embodiment, the DNA ligase inhibitor is a human DNA ligase inhibitor. Herein disclosed the DNA ligase inhibitor is L189, SCR7, SCR7 pyrazine, Rabeprazole or U73122. In an embodiment, the DNA ligase inhibitor is one or more of L189, SCR7, SCR7 pyrazine, Rabeprazole and U73122. In an embodiment, the DNA ligase inhibitor is L189 or SCR7. In an embodiment, the DNA ligase inhibitor is L189. In an embodiment, the DNA ligase inhibitor is SCR7.

In an embodiment, the PARP inhibitor is a PARP-1 inhibitor. In an embodiment, the PARP inhibitor is Talazoparib, Veliparib, Olaparib, Niraparib or Rucaparib. In an embodiment, the PARP inhibitor is one or more of Talazoparib, Veliparib, Olaparib, Niraparib and Rucaparib. In an embodiment, the PARP inhibitor is Talazoparib or Olaparib. In an embodiment, the PARP inhibitor is Talazoparib. In an embodiment, the PARP inhibitor is Olaparib.

In an embodiment, the DNA ligase inhibitor is L189 or SCR7 and the PARP inhibitor is Talazoparib or Olaparib. In an embodiment, the DNA ligase inhibitor is L189 and the PARP inhibitor is Olaparib. In an embodiment, the DNA ligase inhibitor is L189 and the PARP inhibitor is Talazoparib. In an embodiment, the DNA ligase inhibitor is SCR7 and the PARP inhibitor is Olaparib. In an embodiment, the DNA ligase inhibitor is SCR7 and the PARP inhibitor is Talazoparib.

In an embodiment, the ratio of the DNA ligase inhibitor to the PARP inhibitor is selected from any one of 1000:1 to 1:1000, 500:1 to 1:500, 100:1 to 1:100, 50:1 to 1:50, 20:1 to 1:20, 10:1 to 1:10, 5:1 to 1:5, 2:1 to 1:2, 1:1.5 to 1.5:1 and 1:1. In an embodiment, the DNA ligase inhibitor is present in a greater molar quantity than the PARP inhibitor. In an embodiment, the DNA ligase inhibitor is present in a lower molar quantity than the PARP inhibitor.

Herein disclosed there is provided a pharmaceutical combination comprising the combination according to the first aspect of the invention, and/or the embodiments thereof, for use as a medicament.

In an embodiment, the pharmaceutical combination is for use in the treatment of cancer, wherein the cancer is selected from lung cancer, breast cancer, pancreatic cancer, womb (uterus) cancer, melanoma cancer or colorectal cancer.

In an embodiment, the pharmaceutical combination is for use in the treatment of lung cancer, wherein the lung cancer is selected from small cell lung carcinoma, combined small cell carcinoma, adenocarcinoma, squamous cell carcinoma, large cell carcinoma, undifferentiated non-small cell lung cancer, mesothelioma or neuroendocrine tumours. In an embodiment, the pharmaceutical combination is for use in the treatment of breast cancer, wherein the breast cancer is selected from metastatic breast cancer, ductal carcinoma in situ (DCIS), lobular carcinoma in situ (LCIS), invasive ductal breast cancer, invasive lobular breast cancer, inflammatory breast cancer, triple negative breast cancer, medullary carcinoma, tubular carcinoma, mucinous carcinoma, malignant Phyllodes tumour or Paget's disease. In an embodiment, the pharmaceutical combination is for use in the treatment of pancreatic cancer, wherein the pancreatic cancer is selected from pancreatic ductal adenocarcinoma (PDAC), squamous cell carcinoma, adenosquamous carcinoma, acinar cell carcinoma, solid pseudopapillary neoplasm, pancreatoblastoma, Benign precancerous lesions or pancreatic neuroendocrine tumours. In an embodiment, the pharmaceutical combination is for use in the treatment of womb (uterus) cancer, wherein the womb cancer is selected from endometrial adenocarcinoma, adenosquamous carcinoma, papillary serous carcinoma, clear cell carcinoma, uterine sarcoma or cervical cancer. In an embodiment, the pharmaceutical combination is for use in the treatment of melanoma cancer, wherein the melanoma cancer is selected from superficial spreading melanoma, nodular melanoma, lentigo maligna melanoma, acral lentiginous melanoma, ocular melanoma, mucosal lentiginous melanoma, demoplastic melanoma, amelonotic melanoma, cutaneous melanoma or anorectal melanoma. In an embodiment, the pharmaceutical combination is for use in the treatment of colorectal cancer, wherein the colorectal cancer is selected from bowel cancer, colorectal adenocarcinoma, gastrointestinal carcinoid tumours, primary colorectal lymphomas, gastrointestinal stromal tumours, mucinous tumours, signet ring tumours or leiomyosarcomas. In an embodiment, the pharmaceutical combination is for use in the treatment of cancer, wherein the cancer is a drug resistant cancer. In an embodiment, the pharmaceutical combination is for use in the treatment of skin cancer, wherein the skin cancer is selected from basal cell carcinoma, squamous cell carcinoma or melanoma. In an embodiment, the pharmaceutical combination is for use in the treatment of ovarian cancer, wherein the ovarian cancer is selected from epithelial ovarian cancer, stromal ovarian cancer or germ cell ovarian cancer. In an embodiment, the pharmaceutical combination is for use in the treatment of bladder cancer, wherein the bladder cancer is selected from transitional cell (urothelial) bladder cancer, squamous cell bladder cancer, adenocarcinoma, sarcomas or small cell cancer of the bladder.

In an embodiment, the pharmaceutical combination is for use in the treatment of cancer, wherein the drug resistant cancer is selected from breast cancer, lung cancer, pancreatic cancer, melanoma or ovarian cancer.

In an embodiment, the pharmaceutical combination is for use in the treatment of cancer, wherein the drug resistant cancer is lung cancer and the pharmaceutical combination comprises the DNA ligase inhibitor L189 and the PARP inhibitor comprises Olaparib. In an embodiment, the pharmaceutical combination is for use in the treatment of cancer, wherein the drug resistant cancer is lung cancer and the pharmaceutical combination comprises the DNA ligase inhibitor L189 and the PARP inhibitor comprises Talazoparib. In an embodiment, the pharmaceutical combination is for use in the treatment of cancer, wherein the drug resistant cancer is breast cancer and the pharmaceutical combination comprises the DNA ligase comprises SCR7 and the PARP inhibitor comprises Olaparib. In an embodiment, the pharmaceutical combination is for use in the treatment of cancer or drug resistance, wherein the PARP inhibitor comprises a PARP-1 inhibitor that exhibits weaker 'PARP trapping' behaviour such as Olaparib, Niraparib, and Rucaparib.

In an embodiment, the pharmaceutical combination is for use in the treatment of cancer, wherein the cancer is lung cancer and the pharmaceutical combination comprises a DNA ligase IV inhibitor and a PARP inhibitor, wherein the PARP inhibitor comprises Olaparib.

In an embodiment, the pharmaceutical combination is for use in the treatment of cancer, wherein the cancer is pancreatic cancer and the pharmaceutical combination comprises a DNA ligase IV inhibitor and a PARP inhibitor, wherein the PARP inhibitor comprises Olaparib.

In an embodiment, the pharmaceutical combination is for use in the treatment of cancer, wherein the cancer is lung cancer and the pharmaceutical combination comprises a DNA ligase IV inhibitor and a PARP inhibitor, wherein the PARP inhibitor comprises Talazoparib.

In an embodiment, the pharmaceutical combination is for use in the treatment of cancer, wherein the cancer is pancreatic cancer and the pharmaceutical combination comprises a DNA ligase IV inhibitor a PARP inhibitor, wherein the PARP inhibitor comprises Talazoparib.

In an embodiment, the pharmaceutical combination is for use in the treatment of malignancies that have acquired resistance to first-line therapy. In an embodiment, the pharmaceutical combination comprises a PARP inhibitor and a DNA ligase IV inhibitor for use in the treatment of malignancies that have acquired resistance to first-line therapy:.

In an embodiment, the pharmaceutical combination is for use in the treatment of malignancies that possess deficiencies in DNA damage response (DDR) pathways such as mismatch repair (MMR) and single strand annealing (SSA). In an embodiment, the pharmaceutical combination comprises a PARP-1 inhibitor and a DNA ligase IV inhibitor for use in the treatment of malignancies that possess deficiencies in DNA damage response (DDR) pathways such as mismatch repair (MMR) and single strand annealing (SSA). In an embodiment, the pharmaceutical combination is for use in the treatment of non-aggressive or slow-growing cancers. In an embodiment, the pharmaceutical combination comprises a PARP-1 inhibitor and a DNA ligase IV inhibitor for use in the treatment of non-aggressive or slow-growing cancers. In an embodiment, the pharmaceutical combination is for use as an adjunctive therapy alongside a cytotoxic drug or radiotherapy. In an embodiment, the pharmaceutical combination comprises a PARP-1 inhibitor and a DNA ligase IV inhibitor for use as an adjunctive therapy alongside a cytotoxic drug or radiotherapy. Herein disclosed is a pharmaceutical combination comprising a DNA ligase IV inhibitor and a PARP-1 inhibitor in synergistically effective amounts for use as a medicament.

In an embodiment a 'synergistically effective amount' provides a positive 'Excess Over Bliss' (EOB) value. EOB can be defined as the measured kill-rate of Drug A at a certain dosage in combination with Drug B at a certain dosage, subtracted by the theoretical additive kill-rate of both drugs as monotherapies. EOB values vary between -1 and 1 where a negative value indicates antagonism at a given dose pair, and a positive value indicates synergism at a given dose pair. Therefore, synergy is an interaction between two agents that causes the total effect (e.g. cancer cell killing activity) to be greater than the sum of the individual effects of each agent. In an embodiment a synergistically effective amount may be determined by an alternative method of determining synergy such as the highest single agent (HSA) model.

In an embodiment, the pharmaceutical combination is for use in the treatment of cancer, wherein the combination is formulated for oral administration or intravenous administration.

Herein disclosed, there is provided a pharmaceutical combination comprising the combination according to the first aspect of the invention, and/or the embodiments thereof, for the manufacture of a medicament for the treatment of cancer, and optionally drug resistant cancer.

Herein disclosed, there is provided a method for selecting a combination of a DNA ligase I and/or IV inhibitor and a PARP inhibitor for use as a medicament, the method comprising:

a) providing a plurality of combinations of a DNA ligase inhibitor and a PARP inhibitor;
b) for each combination of a DNA ligase inhibitor and a PARP inhibitor, contacting one or more cell with the combination;
c) measuring the synergy of the combination in the one or more cell;
d) calculating a synergy point for each combination;
e) selecting the combination of a DNA ligase inhibitor and a PARP inhibitor with a maximum synergy point.

In an embodiment of the method, the DNA ligase is a DNA ligase IV inhibitor. In an embodiment of the method, the DNA ligase inhibitor is a human DNA ligase inhibitor. Herein disclosed in the method, the DNA ligase inhibitor is L189, SCR7, SCR7 pyrazine, Rabeprazole or U73122. In an embodiment of the method, the DNA ligase inhibitor is L189 or SCR7. In an embodiment of the method, the DNA ligase inhibitor is L189. In an embodiment of the method, the DNA ligase inhibitor is SCR7.

In an embodiment of the method, the PARP inhibitor is a PARP-1 inhibitor. In an embodiment, of the method, the PARP inhibitor is Talazoparib, Veliparib, Olaparib, Niraparib or Rucaparib. In an embodiment of the method, the PARP inhibitor is Talazoparib or Olaparib. In an embodiment of the method, the PARP inhibitor is Talazoparib. In an embodiment of the method, the PARP inhibitor is Olaparib.

In an embodiment of the method, the DNA ligase inhibitor has a binding affinity to DNA ligase between 10-10000 nM. In an embodiment of the method, the DNA ligase inhibitor has a binding affinity to a DNA ligase of approximately 0.01, 0.05, 0.1, 0.5, 1, 10, 100, 1000, 5000 or 10000 nM. In an embodiment of the method, the DNA ligase inhibitor is approximately 0.01 nM. In an embodiment of the method, the DNA ligase inhibitor is approximately 0.05 nM. In an embodiment of the method, the DNA ligase inhibitor is approximately 0.1 nM. In an embodiment of the method, the DNA ligase inhibitor is approximately 0.5 nM. In an embodiment of the method, the DNA ligase inhibitor is approximately 1 nM. In an embodiment of the method, the DNA ligase inhibitor is approximately 10 nM. In an embodiment of the method, the DNA ligase inhibitor is approximately 100 nM. In an embodiment of the method, the DNA ligase inhibitor is approximately 1000 nM. In an embodiment of the method, DNA ligase inhibitor is approximately 5000 nM. In an embodiment of the method, DNA ligase inhibitor is approximately 10000 nM.

In an embodiment, the method further comprises testing the combination of the DNA ligase inhibitor and the PARP inhibitor for its ability to lead to synthetic lethality in vitro.

In an embodiment of the method, the cell line is selected from a lung cancer, breast cancer, pancreatic cancer, womb (uterus) cancer, ovarian cancer, melanoma cancer or colorectal cancer. In an embodiment of the method, the cell line is selected from A549, BT474, BXPC3, CAPAN1, DETROIT562, FADU, MCF7, MDAMB231, MDAMB453, MIAPACA2, NCIH1975, PANC1, UACC-62, OVXF 899L and BXF 1218L. In an embodiment the cell line is A549. In an embodiment the cell line is BT474. In an embodiment the cell line is CAPAN1. In an embodiment the cell line is DETROIT562. In an embodiment the cell line is FADU. In an embodiment the cell line is MCF7. In an embodiment the cell line is MDAMB231. In an embodiment the cell line is MDAMB453. In an embodiment the cell line is MIAPACA2. In an embodiment the cell line is NCIH1975. In an embodiment the cell line is PANC1. In an embodiment the cell line is UACC-62. In an embodiment the cell line is OVXF 899L. In an embodiment the cell line is BXF 1218L.

In an embodiment, the pharmaceutical combination does not comprise a MALAT1 inhibitor or a proton pump inhibitor (PPI). In an embodiment, the pharmaceutical combination comprises only two active agents.

In an embodiment the pharmaceutical combination is a pharmaceutical composition. Therapeutic dosages may be varied depending upon the requirements of the patient, the severity of the condition being treated, and the compound being employed. The magnitude of an effective dose of a compound will vary with the nature of the severity of the condition to be treated and with the particular compound and its route of administration. The daily dose range may be from about 10 $\mu$g to about 30 mg per kg body weight of a human and non-human animal, optionally from about 50 $\mu$g to about 30 mg per kg of body weight of a human and non-human animal, for example from about 50 $\mu$g to about 10 mg per kg of body weight of a human and non-human animal, for example from about 100 $\mu$g to about 30 mg per kg of body weight of a human and non-human animal, for example from about 100 $\mu$g to about 10 mg per kg of body weight of a human and non-human animal and most preferably from about 100 $\mu$g to about 1 mg per kg of body weight of a human and non-human animal.

In an embodiment there is provided a pharmaceutical combination according to the first aspect of the invention, and/or the embodiments thereof, together with at least one pharmaceutically acceptable excipient.

In an embodiment the combination is formulated as a tablet. In an embodiment the combination is formulated as a capsule. In an embodiment the combination is formulated for injection. In an embodiment the injection is intravenous or subcutaneous. In an embodiment the combination is supplied in a sterile buffer solution or as a solid which can be suspended or dissolved in sterile buffer for injection. In an embodiment the pharmaceutically acceptable excipient(s) may be selected from, for example, carriers (e.g. a solid, liquid or semi-solid carrier), adjuvants, diluents (e.g. solid diluents such as fillers or bulking agents; and liquid diluents such as solvents and co-solvents), granulating agents, binders, flow aids, coating agents, release-controlling agents (e.g. release retarding or delaying polymers or waxes), binding agents, disintegrants, buffering agents, lubricants, preservatives, anti-fungal and antibacterial agents, antioxidants, buffering agents, tonicity-adjusting agents, thickening agents, flavouring agents, sweeteners, pigments, plasticizers, taste masking agents, stabilisers or any other excipients conventionally used in pharmaceutical compositions.

The term "pharmaceutically acceptable" as used herein means compounds, materials, compositions, and/or dosage forms which are, within the scope of sound medical judgment, suitable for use in contact with the tissues of a subject (e.g. a human subject) without excessive toxicity, irritation, allergic response, or other problem or complication, commensurate with a reasonable benefit/risk ratio. Each excipient must also be "acceptable" in the sense of being compatible with the other ingredients of the formulation.

The pharmaceutical combination may be a pharmaceutical composition which can be in any form suitable for oral, parenteral, topical, intranasal, intrabronchial, sublingual, ophthalmic, otic, rectal, intra-vaginal, or transdermal administration. Pharmaceutical dosage forms suitable for oral administration include tablets (coated or uncoated), capsules (hard or soft shell), caplets, pills, lozenges, syrups, solutions, powders, granules, elixirs and suspensions, sublingual tablets, wafers or patches such as buccal patches. Tablet compositions can contain a unit dosage of active compound together with an inert diluent or carrier such as a sugar or sugar alcohol, e.g.; lactose, sucrose, sorbitol or mannitol; and/or a non-sugar derived diluent such as sodium carbonate, calcium phosphate, calcium carbonate, or a cellulose or derivative thereof such as microcrystalline cellulose (MCC), methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known in the art. Tablets may be designed to release the drug either upon contact with stomach fluids (immediate release tablets) or to release in a controlled manner (controlled release tablets) over a prolonged period of time or with a specific region of the GI tract.

A pharmaceutical composition typically comprises from approximately 1% (w/w) to approximately 95%, optionally % (w/w) active ingredients and from 99% (w/w) to 5% (w/w) of a pharmaceutically acceptable excipient (for example as defined above) or combination of such excipients. Preferably, the compositions comprise from approximately 20% (w/w) to approximately 90% (w/w) active ingredients and from 80% (w/w) to 10% of a pharmaceutically excipient or combination of excipients. The pharmaceutical compositions may comprise from approximately 1% to approximately 95%, optionally from approximately 20% to approximately 90%, active ingredients. Pharmaceutical compositions according to the invention may be, for example, in unit dose form, such as in the form of ampoules, vials, suppositories, pre-filled syringes, dragées, powders, tablets or capsules.

Tablets and capsules may contain, for example, 0-20% disintegrants, 0-5% lubricants, 0-5% flow aids and/or 0-99% (w/w) fillers/ or bulking agents (depending on drug dose). They may also contain 0-10% (w/w) polymer binders, 0-5% (w/w) antioxidants, 0-5% (w/w) pigments. Slow release tablets would in addition typically contain 0-99% (w/w) release-controlling (e.g. delaying) polymers (depending on dose). The film coats of the tablet or capsule typically contain 0-10% (w/w) polymers, 0-3% (w/w) pigments, and/or 0-2% (w/w) plasticizers. Parenteral formulations typically contain 0-20% (w/w) buffers, 0-50% (w/w) cosolvents, and/or 0-99% (w/w) Water for Injection (WFI) (depending on dose and if freeze dried). Formulations for intramuscular depots may also contain 0-99% (w/w) oils. The pharmaceutical formulation may be presented to a patient in "patient packs" containing an entire course of treatment in a single package, usually a blister pack. The pharmaceutical combination of the invention will generally be presented in unit dosage form and, as such, will typically contain sufficient compounds to provide a desired level of biological activity. For example, a formulation may contain from 1 nanogram to 2 grams of the active ingredients, e.g. from 1 nanogram to 2 milligrams of active ingredients. Within these ranges, particular sub-ranges of compound are 0.1 milligrams to 2 grams of active ingredients (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredients). For oral compositions, a unit dosage form may contain from 1 milligram to 2 grams, more typically 10 milligrams to 1 gram, for example 50 milligrams to 1 gram, e.g. 100 milligrams to 1 gram, of active ingredients. The active ingredients will be administered to a patient in need thereof (for example a human or animal patient) in an amount sufficient to achieve the desired therapeutic effect (effective amount). The precise amounts of compound administered may be determined by a supervising physician in accordance with standard procedures.

Various further aspects and embodiments of the present invention will be apparent to those skilled in the art in view of the present disclosure.

The term "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein. Unless context dictates otherwise, the descriptions and definitions of the features set out above are not limited to any particular aspect or embodiment of the invention and apply equally to all aspects and embodiments which are described.

It will further be appreciated by those skilled in the art that although the invention has been described by way of example with reference to several embodiments. It is not limited to the disclosed embodiments and that alternative embodiments could be constructed without departing from the scope of the invention as defined in the appended claims. The scope of the invention is defined by the claims. Any embodiment not falling under the scope of the claims is provided for information purposes only.

## BRIEF DESCRIPTION OF THE FIGURES

The invention will now be further and more particularly described, by way of example only, and with reference to the accompanying drawings, in which:

Figure 1 shows the final dosages of drugs both as monotherapies and in combination incubated with A549 cells for 120 hours for synergy determination.

Figure 2 shows raw data returned after performing the cell viability assay.

Figure 3 shows the blank corrected cell viability assay raw data.

Figure 4 shows cell viability calculated using blank corrected cell viability assay data.

Figure 5 shows drug synergism between Olaparib and SCR7 in BT474 cells.

Figure 6 shows drug synergism between Talazoparib and L189 in NCIH1975 cells.

Figure 7 shows drug synergism between Olaparib and L189 in NCIH1975 cells.

Figure 8 shows the effect of RNAi mediated knockdown of ligase IV on ligase IV protein levels in various cancer cell lines.

Figure 9 shows the effect of RNAi mediated knockdown of ligase IV on cell viability in various cancer cell lines.

Figure 10 shows synergism between RNAi mediated knockdown of ligase IV and Talazoparib in NCIH1975 cells.

Figure 11 shows synergism between RNAi mediated knockdown of ligase IV and Olaparib in NCIH1975 cells.

Figure 12 shows synergism between RNAi mediated knockdown of ligase IV and Olaparib in A549 cells.

Figure 13 shows synergism between RNAi mediated knockdown of ligase IV and Olaparib in Capan-1 cells.

Figure 14 shows synergism between RNAi mediated knockdown of ligase IV and Talazoparib in Capan-1 cells.

Figure 15 shows a 96-well plate map detailing the final dosages of Olaparib and Talazoparib incubated with siRNA transfected cells of a given cell line for HSA synergy determination.

Figure 16 shows 5x5 combination plots of Olaparib and S3766-1-X dosages in UACC-62 cancer cells, showing cancer cell kill-rate and Bliss synergy scores.

Figure 17 shows 5x5 combination plots of Olaparib and S3766-1-X dosages in OVXF 899L cancer cells, showing cancer cell kill-rate and Bliss synergy scores.

Figure 18 shows the LigIV protein binding activity of S37766-1-X.

## DETAILED DESCRIPTION OF THE INVENTION

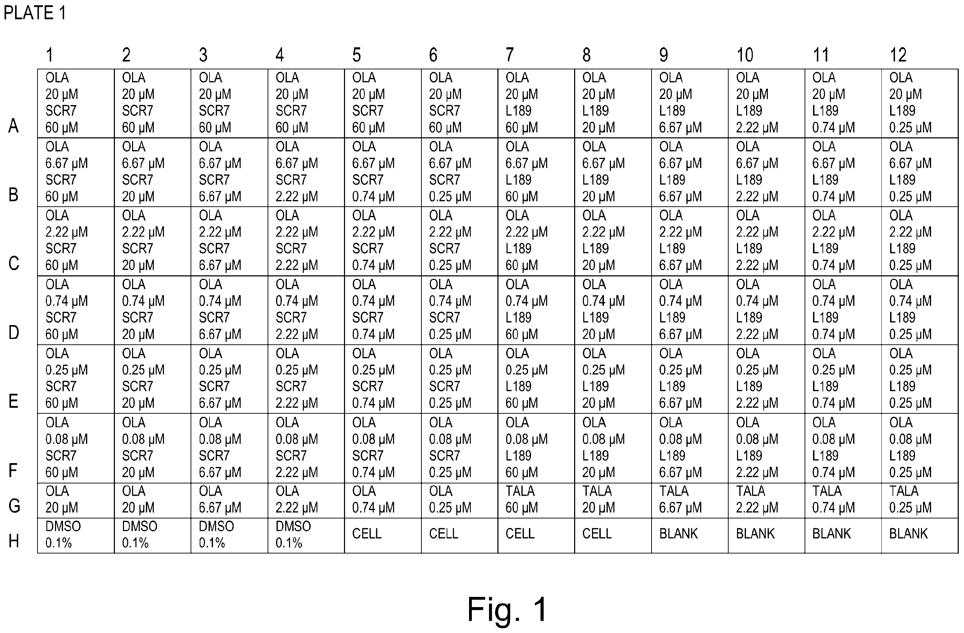

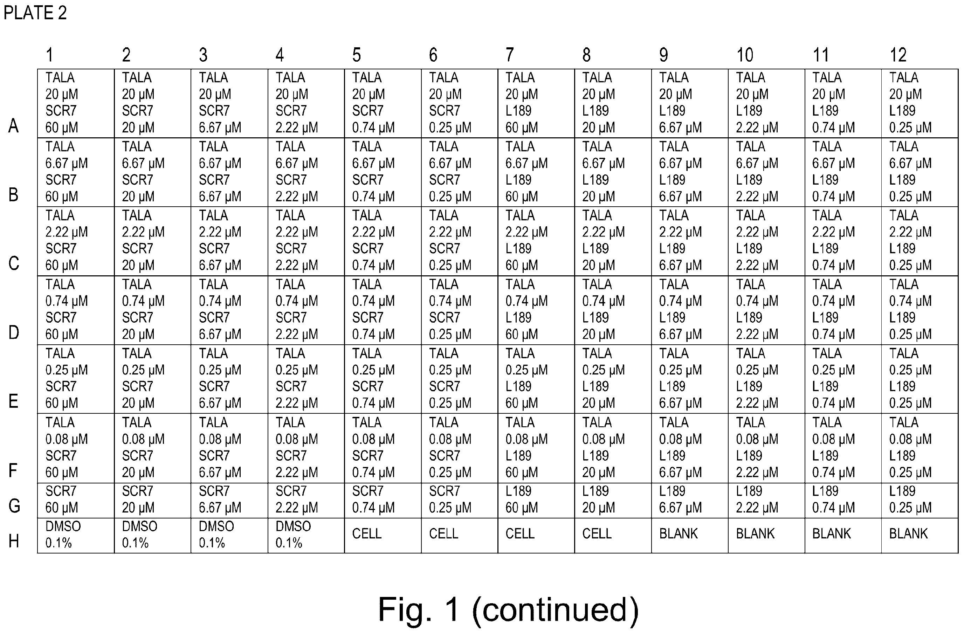

Figure 1 shows a 96-well plate map (**PLATE 1**) where Olaparib (**OLA**) ranges from 20 $\mu$M to 0.08 $\mu$M from rows A to F, SCR7 ranges from 60 $\mu$M to 0.25 $\mu$M from columns 1 to 6, and L189 ranges from 60 $\mu$M to 0.25 $\mu$M from columns 7 to 12. OLA as a monotherapy ranges from 20 $\mu$M to 0.08 $\mu$M from wells G1 to G6, whilst Talazoparib (**TALA**) as a monotherapy ranges from 20 $\mu$M to 0.08 $\mu$M from wells G7 to G12. As shown in Figure 1, there is shown a second 96-well plate map (**PLATE 2**) where TALA ranges from 20 $\mu$M to 0.08 $\mu$M from rows A to F, SCR7 ranges from 60 $\mu$M to 0.25 $\mu$M from columns 1 to 6, and L189 ranges from 60 $\mu$M to 0.25 $\mu$M from columns 7 to 12. SCR7 as a monotherapy ranges from 60 $\mu$M to 0.25 $\mu$M from wells G1 to G6, whilst L189 as a monotherapy ranges from 60 $\mu$M to 0.25 $\mu$M from wells G7 to G12. 3-fold serial dilutions were performed to achieve the dosage ranges in both plates. Wells H1 to H4 are DMSO controls, wells H5 to H8 are untreated cell controls, and wells H9 to H12 are blanks; this is the case for both plates.

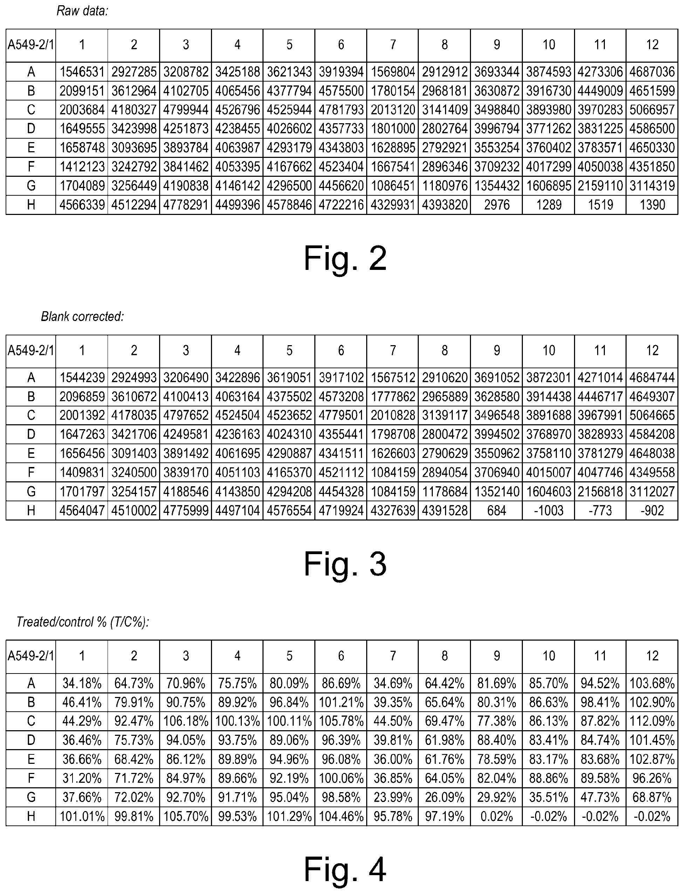

Figure 2 shows a 96-well plate map which presents raw data returned after performing the CellTiter-Glo® luminescent cell viability assay uniformly across all wells of a 96-well plate comprising A549 cells in culture and the drug dosages outlined in PLATE 1 in Figure 1. The drug dosages outlined in PLATE 1 of Figure 1 were incubated with A549 cells for 120 hours before performing the cell viability assay. The data obtained from Figure 1, PLATE 2 were measured, but this data are not shown in Figure 2.

Figure 3 shows a 96-well plate map which presents the raw data returned after performing the CellTiter-Glo® luminescent cell viability assay uniformly across all wells of a 96-well plate comprising A549 cells in culture and the drug dosages outlined in PLATE 1 of Figure 1 that has been blank corrected. Wells H9 to H12 in PLATE 1 of Figure 1 are blanks containing only cell culture medium without cells, Figure 3 shows the raw values subtracted by the mean of all blank readings. Blank corrected repeat measurements were also recorded for A549 cells incubated with the drug dosages outlined in PLATE 2 of Figure 1, but this data is not shown in Figure 3.

Figure 4 shows a 96-well plate map which presents cell viability data for A549 cells after incubation with the dosages outlined in PLATE 1 of Figure 1 where cell viability is calculated as described in example 1. Cell viability was also calculated, as described in example 1, for A549 cells incubated with the drug dosages outlined in PLATE 2 of Figure 1, but this data is not shown in Figure 4.

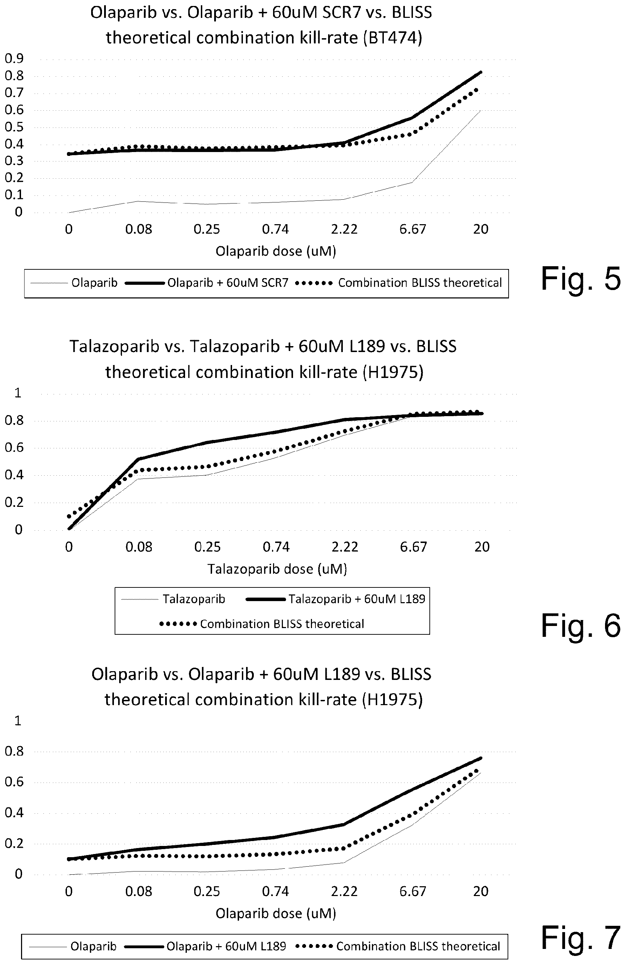

Figure 5 shows a graph with kill-rate on the Y-axis and Olaparib dose on the X-axis showing the kill-rate of Olaparib as a monotherapy (grey), the theoretical additive kill-rate of Olaparib + 60 $\mu$M) SCR7 (dashed line), and the kill-rate of Olaparib in combination with 60 $\mu$M SCR7 (black). To an individual skilled in the art, it can be seen that where Olaparib is at 6.67 $\mu$M in combination with 60 $\mu$M SCR7, there is synergy in BT474 cells.

Figure 6 shows a graph with kill-rate on the Y-axis and Talazoparib dose on the X-axis showing the kill-rate of Talazoparib as a monotherapy (grey), the theoretical additive kill-rate of Talazoparib + 60 $\mu$M L189 (dashed line), and the kill-rate of Talazoparib in combination with 60 $\mu$M L189 (black). To an individual skilled in the art, it can be seen that where Talazoparib is at 0.25 $\mu$M in combination with 60 $\mu$M L189, there is synergy in NCIH1975 cells.

Figure 7 shows a graph with kill-rate on the Y-axis and Olaparib dose on the X-axis showing the kill-rate of Olaparib as a monotherapy (grey), the theoretical additive kill-rate of Olaparib + 60 $\mu$M L189 (dashed line), and the kill-rate of Olaparib in combination with 60 $\mu$M L189 (black). To an individual skilled in the art, it can be seen that where Olaparib is at 6.67 $\mu$M in combination with 60 $\mu$M L189, there is synergy in NCIH1975 cells.

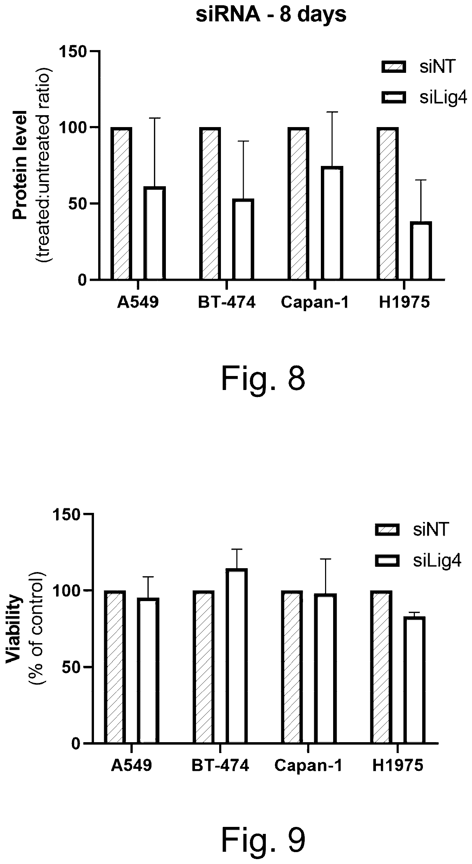

Figure 8 shows a bar chart with ligase IV protein level plotted against the following cancer cell lines: A549, BT-474, Capan-1 and H1975. For each cancer cell line, the lighter bar represents the level of ligase IV protein in cells treated with small interfering non-targeted RNA (siNT) for 8 days, whilst the darker bar represents cells treated with small interfering RNA targeted to ligase IV mRNA for 8 days causing ligase IV protein knockdown. Small interfering non-targeted RNA is used as a control. Ligase IV protein is knocked down in each cell line after treatment with siRNA targeted to ligase IV mRNA compared to controls.

Figure 9 shows a bar chart with cell viability plotted against the following cancer cell lines: A549, BT-474, Capan-1 and H1975. For each cancer cell line, the lighter bar represents cell viability for cells treated with small interfering non-targeted RNA (siNT), whilst the darker bar represents cell viability for cells treated with small interfering RNA targeted to ligase IV mRNA causing ligase IV protein knockdown. Small interfering non-targeted RNA is used as a control. Ligase IV knockdown does not significantly affect cell viability in any cancer cell line compared to controls.

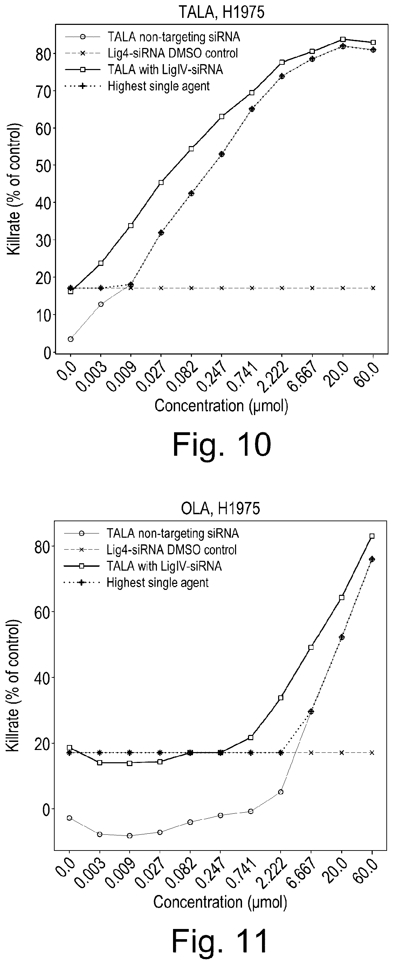

Figure 10 shows a graph with H1975 cell kill-rate on the Y-axis and Talazoparib dose on the X-axis showing the kill-rate of Talazoparib as a monotherapy (circles), the kill-rate of LigIV-siRNA as a monotherapy (x marks), the theoretical HSA kill-rate of Talazoparib + LigIV-siRNA (crosses), and the kill-rate of Talazoparib in combination with LigIV-siRNA (squares). Where LigIV-siRNA is used as a monotherapy, a DMSO control is used instead of Talazoparib. Where Talazoparib is used as a monotherapy, a small interfering non-targeted RNA control is used instead of LigIV-siRNA. To an individual skilled in the art, it can be seen that Ligase IV protein knockdown in combination with Talazoparib is synergistic in H1975 cells across the entire concentration range of Talazoparib ($\mu$M) tested.

Figure 11 shows a graph with H 1975 cell kill-rate on the Y-axis and Olaparib dose on the X-axis showing the kill-rate of Olaparib as a monotherapy (circles), the kill-rate of LigIV-siRNA as a monotherapy (x marks), the theoretical HSA kill-rate of Olaparib + LigIV-siRNA (crosses), and the kill-rate of Olaparib in combination with LigIV-siRNA (squares). Where LigIV-siRNA is used as a monotherapy, a DMSO control is used instead of Olaparib. Where Olaparib is used as a monotherapy, a small interfering non-targeted RNA control is used instead of LigIV-siRNA. To an individual skilled in the art, it can be seen that Ligase IV protein knockdown in combination with Olaparib is synergistic in H1975 cells within the range of 0.247 - >60 $\mu$mol Olaparib.

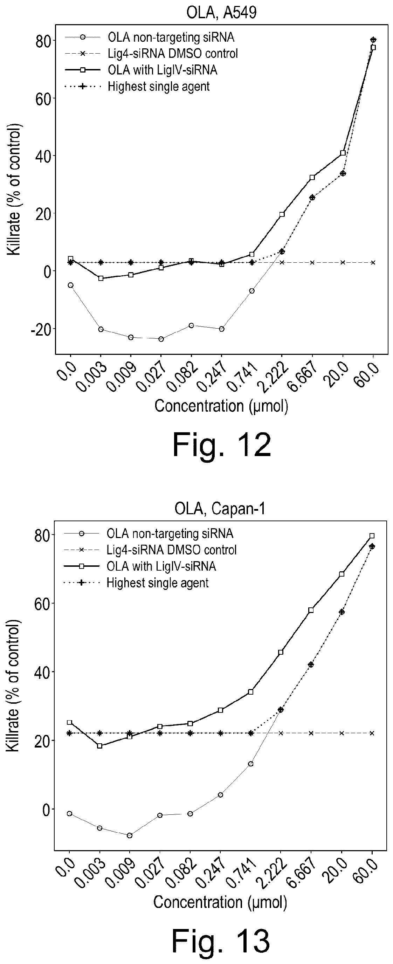

Figure 12 shows a graph with A549 cell kill-rate on the Y-axis and Olaparib dose on the X-axis showing the kill-rate of Olaparib as a monotherapy (circles), the kill-rate of LigIV-siRNA as a monotherapy (x marks), the theoretical HSA kill-rate of Olaparib + LigIV-siRNA (crosses), and the kill-rate of Olaparib in combination with LigIV-siRNA (squares). Where LigIV-siRNA is used as a monotherapy, a DMSO control is used instead of Olaparib. Where Olaparib is used as a monotherapy, a small interfering non-targeted RNA control is used instead of LigIV-siRNA. To an individual skilled in the art, it can be seen that Ligase IV protein knockdown in combination with Olaparib is synergistic in A549 cells within the range of 0.741 - 20 $\mu$mol Olaparib.

Figure 13 shows a graph with Capan-1 cell kill-rate on the Y-axis and Olaparib dose on the X-axis showing the kill-rate of Olaparib as a monotherapy (circles), the kill-rate of LigIV-siRNA as a monotherapy (x marks), the theoretical HSA kill-rate of Olaparib + LigIV-siRNA (crosses), and the kill-rate of Olaparib in combination with LigIV-siRNA (squares). Where LigIV-siRNA is used as a monotherapy, a DMSO control is used instead of Olaparib. Where Olaparib is used as a monotherapy, a small interfering non-targeted RNA control is used instead of LigIV-siRNA. To an individual skilled in the art, it can be seen that Ligase IV protein knockdown in combination with Olaparib is synergistic in Capan-1 cells within the range of 0.027 - >60 $\mu$mol Olaparib.

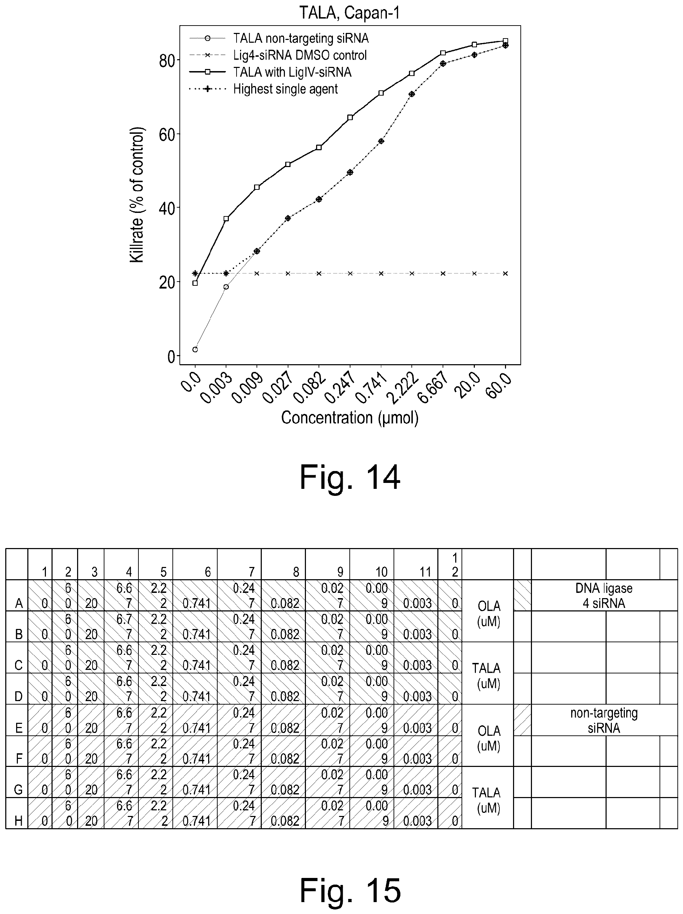

Figure 14 shows a graph with Capan-1 cell kill-rate on the Y-axis and Talazoparib dose on the X-axis showing the kill-rate of Talazoparib as a monotherapy (circles), the kill-rate of LigIV-siRNA as a monotherapy (x marks), the theoretical HSA kill-rate of Talazoparib + LigIV-siRNA (crosses), and the kill-rate of Talazoparib in combination with LigIV-siRNA (squares). Where LigIV-siRNA is used as a monotherapy, a DMSO control is used instead of Talazoparib. Where Talazoparib is used as a monotherapy, a small interfering non-targeted RNA control is used instead of LigIV-siRNA. To an individual skilled in the art, it can be seen that Ligase IV protein knockdown in combination with Talazoparib is synergistic in Capan-1 cells across the entire concentration range of Talazoparib ($\mu$M) tested.

Figure 15 shows a 96 well plate map where Olaparib ranges from 60 $\mu$M to 0.003 $\mu$M from A2 to A11, B2 to B11, E2 to E11 and F2 to F11. Talazoparib ranges from 60 $\mu$M to 0.003 $\mu$M from C2 to C11, D2 to D11, G2 to G11 and H2 to H11. Rows A to D are seeded with cells treated with LigIV-siRNA whilst rows E to H are seeded with cells treated with small interfering non-targeted RNA. 3-fold serial dilutions were performed to achieve the dosage ranges. Columns 1 and 12 are DMSO controls.

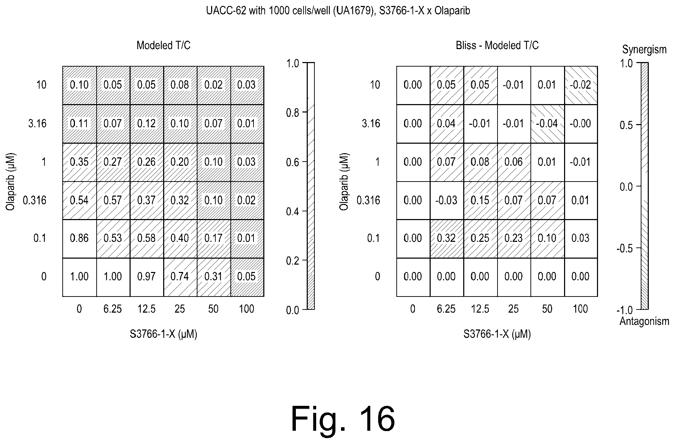

Figure 16 (Left) shows a 5x5 combination plot with Olaparib dose ascending from 0 to 10 $\mu$M from the bottom row upwards, and S3766-1-X dose ascending from 0 to 100 $\mu$M from columns left to right. The leftmost column represents Olaparib as a monotherapy and bottom row represents S3766-1-X as a monotherapy. Shading for each dose pair represents UACC-62 cancer cell kill rate as calculated in example 1. (Right) shows a 5x5 combination plot with Olaparib dose ascending from 0 to 10 $\mu$M from the bottom row upwards, and S3766-1-X dose ascending from 0 to 100 $\mu$M from columns left to right. The leftmost column represents Olaparib as a monotherapy and bottom row represents S3766-1-X as a monotherapy. Shading in this plot indicates synergy (shading with diagonal lines which ascend from left to right) according to the bliss independence model of synergy determination. The inhibitor(s) where incubated with UACC-62 cells for seven days. The person skilled in the art would understand from this that the combination of Olaparib and S3766-1-X is synergistic as an anticancer treatment in the UACC-62 cancer cell line after 7 days of treatment, particularly at lower concentrations of Olaparib.

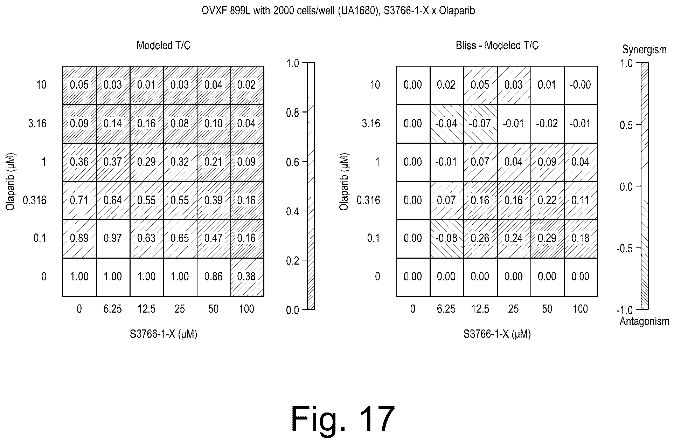

Figure 17 (Left) shows a 5x5 combination plot with Olaparib dose ascending from 0 to 10 $\mu$M from the bottom row upwards, and S3766-1-X dose ascending from 0 to 100 $\mu$M from columns left to right. The leftmost column represents Olaparib as a monotherapy and bottom row represents S3766-1-X as a monotherapy. Shading for each dose pair represents OVXF 899L cancer cell kill rate as calculated in example 1. (Right) shows a 5x5 combination plot with Olaparib dose ascending from 0 to 10 $\mu$M from the bottom row upwards, and S3766-1-X dose ascending from 0 to 100 $\mu$M from columns left to right. The leftmost column represents Olaparib as a monotherapy and bottom row represents S3766-1-X as a monotherapy. Shading in this plot indicates synergy (shading with diagonal lines which ascend from left to right) according to the bliss independence model of synergy determination. The inhibitor(s) where incubated with OVXF 899L cells for seven days. The person skilled in the art would understand from this that the combination of Olaparib and S3766-1-X is synergistic as an anticancer treatment in the OVXF 899L cancer cell line after 7 days of treatment, particularly at lower concentrations of Olaparib.

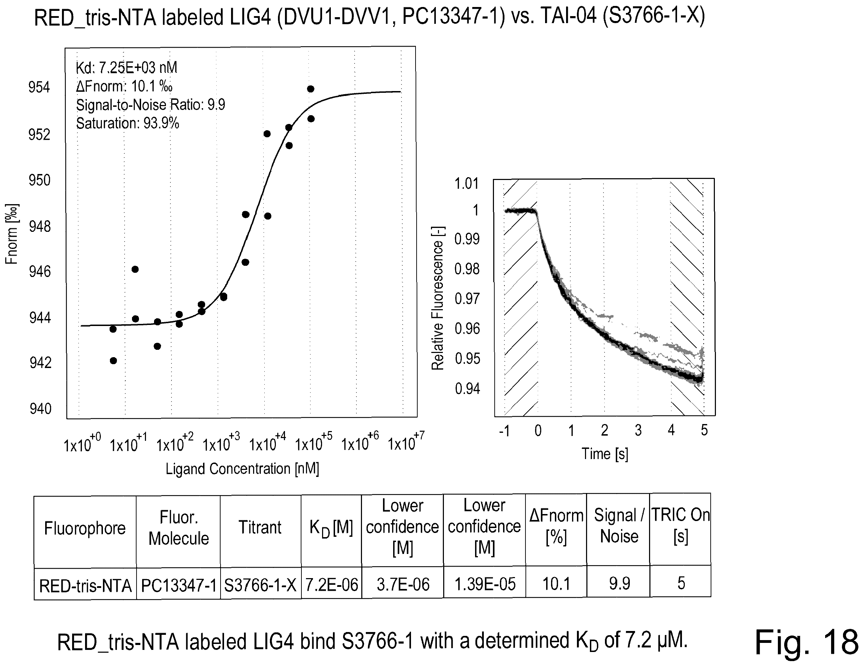

Figure 18 shows LigIV binding activity of S3766-1-X. TRIC traces for 12 S3766-1-X concentration points are transformed into a dose-response curve (left) which is modelled to yield the steady-state affinity of the molecular interaction between S3766-1-X and LigIV protein. S3766-1-X binds LigIV protein with a KD of 7.2 $\mu$M.

## METHODS AND EXAMPLES

### Example 1 - Investigating the combination of DNA ligase IV and PARP inhibitors on A549 lung cancer cells

Cell Culture

A549 cells (Hungarian Academy of Sciences - Semmelweis University -Pathobiochemistry Research Group) were cultured in a 25 cm$^3$ flask with 5 mL cell culture medium (Dulbecco's Modified Eagle's Medium (DMEM) supplemented with 10% fetal bovine serum and MycoZap™ Plus-CL) in a humidified $CO_2$ incubator at 37 °C. To seed the cells, cell culture medium was removed from the flask and the cells were washed with 2 ml PBS, then 1 ml trypsine-EDTA solution was added and the cells were incubated in the $CO_2$ incubator. When the cells detached they were re-suspended in 3 ml cell culture medium to inhibit trypsin and centrifuged with 150xg for 4 minutes at room temperature. The supernatant was discarded and the cells were re-suspended in 5 ml cell culture medium and the exact cell number was determined using hemocytometer after Trypan blue staining. Cells were plated into 2 sterile white 96-well plates at a density of 1000 cells/well in 50 $\mu$l cell culture medium and were incubated for 24 hours at 37°C in $CO_2$ incubator. After 24 hours the cells were treated with the indicated compounds, giving the final concentrations as shown in Figure 1, and a final volume of 100 $\mu$l. The plates were incubated in a $CO_2$ incubator at 37°C for 120 hours.

Cell Viability Assay

After the incubation, cell viability was measured with the CellTiter-Glo® luminescent cell viability assay (Promega, Madison, WI, USA). CellTiter Glo™ reagent was added to each well according to the manufacturer's description. The luminescence signal was recorded using a microplate reader (BioTek Synergy 2 Multi-Mode Reader, Winooski, VT, USA). Raw data was blank corrected and viability was calculated as follows:

$$\text{Treated/control\% (T/C\%)}$$

$$Cell\ kill\text{-}rate = 1 - (cell\ viability)$$

Dose response curves (using non-linear regression model) were generated and $IC_{50}$ values were determined by Graph Pad Prism 5.02 software.

Determination of Synergy

Synergy was calculated for each dose pair in all combinations using the Bliss independence model. For each dose-pair in the grid the measured cell viability was converted to effect:

$$\sigma_{ij,\ measured} = 1 - \varepsilon_{ij}$$

Here $\sigma$ is the effect, $\varepsilon$ is the viability, and i and j are the doses of compounds.
Next, theoretical additive effects were determined:

$$\sigma_{ij,\ theor} = \sigma_{0j} + \sigma_{i0} - \sigma_{0j} {}^* \sigma_{i0}$$

For each dose-pair the Bliss synergy score, also known as 'Excess over Bliss' (EOB) was calculated in the following way:

$$EOB_{i,j} = \sigma_{ij,\ measured} - \sigma_{ij,\ theor}$$

Here a positive EOB indicates synergism, and a negative EOB indicates antagonism at a given dose pair; the EOB value ranges between (-1 to 1).

**Results**

The invention is now demonstrated with reference to the following experimental data.
Cell viability, kill-rate, and synergy were all calculated using the averages across the multiple measurements collected.

Table 1. BT474 cell line - synergistic dose combination of Olaparib and SCR7

| BT474 cell line | | | | |
|---|---|---|---|---|
| Drug Therapy | Viability / $\varepsilon_{ij}$ | Combination Kill Rate / $\sigma_{ij}$, measured | Theoretical Kill Rate / $\sigma_{ij}$, theor | Excess Over Bliss / EOBij |
| Olaparib 6.67 $\mu$M | 0.825 | | 0.46 | |
| SCR7 60 $\mu$M | 0.653 | | | |
| Olaparib 6.67 $\mu$M and SCR7 60 $\mu$M | 0.443 | 0.557 | | 0.097 |

Table 2. NCIH1975 cell line - synergistic dose combination of Talazoparib and L189

| NCIH1975 cell line | | | | |
|---|---|---|---|---|
| Drug Therapy | Viability/ $\varepsilon_{ij}$ | Combination Kill Rate / $\sigma_{ij}$, measured | Theoretical Kill Rate / $\sigma_{ij}$, theor | Excess Over Bliss / EOBij |
| Talazoparib 0.25 $\mu$M | 0.597 | | 0.46 | |
| L189 60 $\mu$M | 0.898 | | | |
| Talazoparib 0.25 $\mu$M and L189 60 $\mu$M | 0.357 | 0.643 | | 0.183 |

Table 3. NCIH1975 cell line - synergistic dose combination of Olaparib and L189

| NCIH1975 cell line | | | | |
|---|---|---|---|---|
| Drug Therapy | Viability/ $\varepsilon_{ij}$ | Combination Kill Rate / $\sigma_{ij}$, measured | Theoretical Kill Rate / $\sigma_{ij}$, theor | Excess Over Bliss / EOBij |
| Olaparib 6.67 $\mu$M | 0.678 | | 0.39 | |
| L189 60 $\mu$M | 0.897 | | | |
| Olaparib 6.67 $\mu$M and L189 60 $\mu$M | 0.446 | 0.554 | | 0.164 |

**Example 2 - RNAi Mediated Knockdown of Ligase IV**

The invention has been further demonstrated using RNA interference methods to inhibit ligase IV instead of small molecule compounds. Small interfering RNAs (siRNAs) operate within the RNA interference pathway; siRNAs have been used in this disclosure as RNA interference method of ligase IV inhibition. LigIV-siRNA selectively silences the ligase IV gene and knocks down ligase IV protein levels with no off-target activity (Fig. 8). Inhibitors such as Olaparib and Talazoparib are potent and selective PARP inhibitors and have been used in combination with LigIV-siRNA in various cancer cell lines; synergy has been determined with high confidence which validates the concept of the invention. LigIV-siRNA is synergistic in combination with Talazoparib in H1975 cells (Fig. 10) and Capan-1 cells (Fig. 14). LigIV-siRNA is also synergistic in combination with Olaparib in H1975 cells (Fig. 11), A549 cells (Fig. 12) and Capan-1 cells (Fig. 13). In an embodiment, another means of ligase IV inhibition known in the art may be used for the purpose of the invention.

Transfection

The following cancer cell lines A549, BT474, Capan-1 and H1975 were cultured using standard protocols known in the art - for reference see example 1. The cells were seeded onto a multi-well plate and incubated with transfection solution for 24 hours. For each cell line, cells were transfected with LigIV-siRNA whilst a separate sample was transfected with small interfering non-targeted RNA as a control.

Gel Electrophoresis

The transfected cells were lysed and the supernatant was recovered for each condition (LigIV-siRNA treated and siNT treated) and cell line. The Bradford method was then used to measure total protein content for subsamples of each sample such that protein levels could be normalised before western blot analysis. The samples for each condition and cell line were then run on a polyacrylamide gel (SDS-PAGE) for 15 minutes at 80 V and 1.5-2 hours at 130 V to separate proteins according to their molecular mass. Proteins on the gels were transferred into polyvinylidene difluoride (PVDF) membranes.

Western Blotting

The PVDF membranes were blocked and then incubated in primary antibody solution (e.g. DNA Ligase IV (D5N5N) Rabbit mAb (Cell Signalling 14649S)) overnight. The membranes were then incubated in secondary antibody solution for 1 hour before being developed and imaged. Ligase IV protein levels were then normalised to $\beta$-Actin and quantified for each cell line and each condition. Ligase IV protein levels were significantly knocked down after transfection with LigIV-siRNA in each cell line compared to controls (Fig. 8). Moreover, ligase IV protein knockdown as a monotherapy had no significant effect on cell viability in each cell line compared to controls (Fig. 9).

RNAi Cell Viability Assay

After gene silencing, cells for each cell line - both LigIV-siRNA and siNT treated - were plated into sterile white 96-well plates (one 96-well plate per cell line) and were incubated for 24 hours at 37°C in $CO_2$ incubator. After 24 hours the cells were treated with Olaparib or Talazoparib at the indicated concentrations for 5 days treatment time (Fig. 15) in duplicate - 3-fold serial dilutions were made. Untreated control wells were supplemented with DMSO containing medium to 0.12% final concentration. After incubation, CellTiter Glo™ reagent was added to each well according to the manufacturer's description. The luminescent signal was recorded and cell viability was calculated - for reference see example 1. Synergy was then determined according to the HSA synergy model.

According to the HSA model, synergy is observed where the measured kill-rate for a given PARP inhibitor dosage and LigIV-siRNA pair is greater than the theoretical HSA kill-rate for that same PARP inhibitor dosage and LigIV-siRNA pair in a given cell line. HSA (theoretical) is the maximal single agent effect at a given dose pair.

## Example 3 - S3766-1-X in combination with Olaparib

The inventors of the present disclosure have demonstrated that the pharmaceutical combination of a PARP inhibitor and a LigIV inhibitor is synergistic as an anticancer treatment for various lineages of cancer including varieties of breast cancer, lung cancer and pancreatic cancer. The inventors have also demonstrated that the pharmaceutical combination of a PARP inhibitor and a LigIV inhibitor is synergistic as an anticancer treatment for other varieties of cancer including melanoma and ovarian cancer. Compound "S3766-1-X", a derivative of the LigIV inhibitor "SCR7" was tested in combination with Olaparib at different dosage pairs for cancer cell killing activity in both a melanoma cancer cell line (UACC-62) (Fig. 16) and an ovarian cancer cell line (OVXF 899L) (Fig. 17). The cancer cell line "OVXF 899L" is a PDX (Patient Derived Xenograft) cancer cell line. For synergy determination, the same methodology was used as is set forth in example 1. The person skilled in the art would understand from this that the pharmaceutical combination of Olaparib and S3766-1-X is synergistic as an anticancer treatment for both UACC-62 cancer cells and OVXF 899L cancer cells, particularly at lower dosages of Olaparib. These findings highlight the broad utility of such a pharmaceutical combination as an effective anticancer therapy for a wide variety of clinical indications.

## Example 4 - LigIV binding assay

LigIV binding activity of S3766-1-X was measured on a NanoTemper Dianthus NT.23PicoDuo device. The Dianthus technology is based on the TRIC effect (temperature-related intensity-change of fluorescent molecules). These fluorescence changes are very sensitively dependent on the electronic microenvironment of the fluorescent molecule. Thus, if a fluorescent dye is coupled to a target molecule (protein); its TRIC effect will react to changes by ligand binding or by ligand binding-induced conformational changes. Mixes of the fluorescently-labeled target molecule and increasing concentrations of the ligand molecule (1000 $\mu$ - 5.6 nM for the S3766-1-X titrant) are placed in 12 adjacent wells. An infrared laser is used to generate a precise temperature gradient while an LED is used for the excitation of fluorescent molecules in the wells. The laser-induced temperature gradient leads to the TRIC-based changes of the fluorescence of the labeled target molecule, which is recorded by fluorescence optics. Different TRIC-responses are obtained for different states of the target molecule, i.e. ligand-unbound and ligand-bound states.

The comparison of the TRIC traces for all 12 ligand-concentration points reveals that there is a change in the TRIC response that correlates with the ligand concentration. The TRIC traces can finally be transformed to a dose-response curve, which can be fitted to various models to yield the steady-state affinity of the analysed molecular interaction - a $K_D$ of 7.2 $\mu$M in the case of S3766-1-X (Fig. 18). The person skilled in the art would understand from this that S3766-1-X binds with high affinity to its intended target - LigIV protein. For assay conditions see table 4.

**Table 4. TRIC assay conditions: RED-tris-NTA labeled LIG4 vs. compound +/- dsDNA**

| | |
|---|---|
| Fluor. Molecule | 50 nM LigIV (DVU1-DVV1, PC13347-1) |
| Fluorophore | 25 nM RED-tris-NTA 1st generation |
| Labeling conditions | Labeling buffer: Assay Buffer 100 nM protein/50 nM dye; Incubation time: 30 min |
| Instrument | Dianthus NT.23PicoDuo |
| Measuring parameter | LED Power: 15 %; MST settings: 1 - 5 -1 (s) (initial fluorescence - MST on time - back-diffusion), Duplicates |
| Assay buffer | 20 nM Hepes pH 7.5, 30 mM NaCl, 10 mM MgCl2, 5mM Glutathione, 0.05% Tween |

## Claims

1. A pharmaceutical combination for use in the treatment of cancer comprising a DNA ligase IV inhibitor and a PARP inhibitor in synergistically effective amounts for simultaneous, separate or sequential administration to a subject in need thereof, wherein the cancer is a drug resistant cancer, wherein the DNA ligase IV inhibitor comprises L189 or SCR7 and the PARP inhibitor comprises Talazoparib or Olaparib.

2. The pharmaceutical combination for use of claim 1, wherein the DNA ligase IV inhibitor comprises a human DNA ligase IV inhibitor.

3. The pharmaceutical combination for use of claim 1 or 2, wherein

the DNA ligase IV inhibitor comprises L189 and the PARP inhibitor comprises Olaparib; or
the DNA ligase IV inhibitor comprises L189 and the PARP inhibitor comprises Talazoparib; or
the DNA ligase IV inhibitor comprises SCR7 and the PARP inhibitor comprises Olaparib; or
the DNA ligase IV inhibitor comprises SCR7 and the PARP inhibitor comprises Talazoparib.

4. The pharmaceutical combination for use in the treatment of cancer of any preceding claim, wherein the cancer is selected from lung cancer, breast cancer, pancreatic cancer, womb (uterus) cancer, ovarian cancer, skin cancer (e.g. melanoma cancer) or colorectal cancer.

5. The pharmaceutical combination for use in the treatment of lung cancer according to claim 4, wherein:

the lung cancer is selected from small cell lung carcinoma, combined small cell carcinoma, adenocarcinoma, squamous cell carcinoma, large cell carcinoma, undifferentiated non-small cell lung cancer, mesothelioma or neuroendocrine tumours; or
the breast cancer is selected from metastatic breast cancer, ductal carcinoma in situ (DCIS), lobular carcinoma in situ (LCIS), invasive ductal breast cancer, invasive lobular breast cancer, inflammatory breast cancer, triple negative breast cancer, medullary carcinoma, tubular carcinoma, mucinous carcinoma, malignant Phyllodes tumour or Paget's disease; or
the pancreatic cancer is selected from pancreatic ductal adenocarcinoma (PDAC), squamous cell carcinoma, adenosquamous carcinoma, acinar cell carcinoma, solid pseudopapillary neoplasm, pancreatoblastoma, benign precancerous lesions or pancreatic neuroendocrine tumours; or
the skin cancer is selected from basal cell carcinoma, squamous cell carcinoma or melanoma; or
the ovarian cancer is selected epithelial ovarian cancer, stromal ovarian cancer and germ cell ovarian cancer.

6. The pharmaceutical combination for use in the treatment of cancer according to any one of claims 1 to 5, wherein the drug resistant cancer is selected from breast, lung or pancreatic cancer.

7. The pharmaceutical combination for use in the treatment of cancer according to any of claims 1 to 3, wherein

the cancer is lung cancer and the pharmaceutical combination comprises the DNA ligase inhibitor L189 and the PARP inhibitor Olaparib, and wherein the lung cancer is drug resistant; or
wherein the cancer is lung cancer and the pharmaceutical combination comprises the DNA ligase inhibitor L189 and the PARP inhibitor Talazoparib, and wherein the lung cancer is drug resistant; or
the cancer is breast cancer and the pharmaceutical combination comprises the DNA ligase inhibitor SCR7 and the PARP inhibitor Olaparib, and wherein the lung cancer is drug resistant; or
the cancer is lung cancer and the pharmaceutical combination comprises the PARP inhibitor Olaparib; or
the cancer is pancreatic cancer and the pharmaceutical combination comprises the PARP inhibitor Olaparib; or
the cancer is lung cancer and the pharmaceutical combination comprises the PARP inhibitor Talazoparib; or
the cancer is pancreatic cancer and the pharmaceutical combination comprises the PARP inhibitor Talazoparib.

8. The pharmaceutical combination for use of any preceding claim, wherein the ratio of the DNA ligase IV inhibitor to the PARP inhibitor is selected from any one of 1000:1 to 1:1000, 500:1 to 1:500, 100:1 to 1:100, 50:1 to 1:50, 20:1 to 1:20, 10:1 to 1:10, 5:1 to 1:5, 2:1 to 1:2, 1:1.5 to 1.5:1 and 1:1.

9. The pharmaceutical combination for use of any preceding claim, wherein the DNA ligase IV inhibitor is present in a greater molar quantity than the PARP inhibitor.

10. The pharmaceutical combination for use of claim 7, wherein the ratio of the DNA ligase IV inhibitor to the PARP inhibitor is selected from any one of 1000:1 to 1:1000, 500:1 to 1:500, 100:1 to 1:100, 50:1 to 1:50, 20:1 to 1:20, 10:1 to 1:10, 5:1 to 1:5, 2:1 to 1:2, 1:1.5 to 1.5:1 and 1:1.

11. The pharmaceutical combination for use of claim 7 or 8, wherein the DNA ligase IV inhibitor is present in a greater molar quantity than the PARP inhibitor.

12. The pharmaceutical combination for use in the treatment of cancer of any preceding claim, wherein the combination is formulated for oral administration or parenteral (e.g. intravenous) administration.

**Patentansprüche**

1. Pharmazeutische Kombination zur Verwendung bei der Behandlung von Krebs, umfassend einen DNA-Ligase-IV-Inhibitor und einen PARP-Inhibitor in synergistisch wirksamen Mengen zur gleichzeitigen, getrennten oder aufeinanderfolgenden Verabreichung an ein Subjekt, das dessen bedarf, wobei der Krebs ein arzneimittelresistenter Krebs ist,
wobei der DNA-Ligase-IV-Inhibitor L189 oder SCR7 umfasst und der PARP-Inhibitor Talazoparib oder Olaparib umfasst.

2. Pharmazeutische Kombination zur Verwendung nach Anspruch 1, wobei der DNA-Ligase-IV-Inhibitor einen menschlichen DNA-Ligase-IV-Inhibitor umfasst.

3. Pharmazeutische Kombination zur Verwendung nach Anspruch 1 oder 2, wobei

der DNA-Ligase-IV-Inhibitor L189 umfasst und der PARP-Inhibitor Olaparib umfasst; oder
der DNA-Ligase-IV-Inhibitor L189 umfasst und der PARP-Inhibitor Talazoparib umfasst; oder
der DNA-Ligase-IV-Inhibitor SCR7 umfasst und der PARP-Inhibitor Olaparib umfasst; oder
der DNA-Ligase-IV-Inhibitor SCR7 umfasst und der PARP-Inhibitor Talazoparib umfasst.

4. Pharmazeutische Kombination zur Verwendung bei der Behandlung von Krebs nach einem der vorhergehenden Ansprüche, wobei der Krebs aus Lungenkrebs, Brustkrebs, Bauchspeicheldrüsenkrebs, Gebärmutterkrebs, Eierstockkrebs, Hautkrebs (z. B. Melanom) oder Kolorektalkrebs ausgewählt ist.

5. Pharmazeutische Kombination zur Verwendung bei der Behandlung von Lungenkrebs nach Anspruch 4, wobei:

der Lungenkrebs aus kleinzelligem Lungenkarzinom, kombiniertem kleinzelligem Karzinom, Adenokarzinom, Plattenepithelkarzinom, großzelligem Karzinom, undifferenziertem nichtkleinzelligem Lungenkrebs, Mesotheliom oder neuroendokrinen Tumoren ausgewählt ist; oder
der Brustkrebs aus metastasiertem Brustkrebs, duktalem Karzinom in situ (DCIS), lobulärem Karzinom in situ (LCIS), invasivem duktalem Brustkrebs, invasivem lobulärem Brustkrebs, entzündlichem Brustkrebs, dreifach negativem Brustkrebs, medullärem Karzinom, tubulärem Karzinom, muzinösem Karzinom, bösartigem Phyllodes-Tumor oder Morbus Paget ausgewählt ist; oder
der Bauchspeicheldrüsenkrebs aus duktalem Adenokarzinom des Pankreas (PDAC), Plattenepithelkarzinom, adenosquamösem Karzinom, Azinuszellkarzinom, solidem pseudopapillärem Neoplasma, Pankreatoblastom, gutartigen präkanzerösen Läsionen oder neuroendokrinen Tumoren der Bauchspeicheldrüse ausgewählt ist; oder
der Hautkrebs aus Basalzellkarzinom, Plattenepithelkarzinom oder Melanom ausgewählt ist; oder
der Eierstockkrebs aus epithelialem Eierstockkrebs, stromalem Eierstockkrebs und Keimzell-Eierstockkrebs ausgewählt ist.

6. Pharmazeutische Kombination zur Verwendung bei der Behandlung von Krebs nach einem der Ansprüche 1 bis 5, wobei der arzneimittelresistente Krebs aus Brust-, Lungen- oder Bauchspeicheldrüsenkrebs ausgewählt ist.

7. Pharmazeutische Kombination zur Verwendung bei der Behandlung von Krebs nach einem der Ansprüche 1 bis 3, wobei

der Krebs Lungenkrebs ist und die pharmazeutische Kombination den DNA-Ligase-Inhibitor L189 und den PARP-Inhibitor Olaparib umfasst und wobei der Lungenkrebs arzneimittelresistent ist; oder
wobei der Krebs Lungenkrebs ist und die pharmazeutische Kombination den DNA-Ligase-Inhibitor L189 und den PARP-Inhibitor Talazoparib umfasst und wobei der Lungenkrebs arzneimittelresistent ist; oder
der Krebs Brustkrebs ist und die pharmazeutische Kombination den DNA-Ligase-Inhibitor SCR7 und den PARP-Inhibitor Olaparib umfasst und wobei der Lungenkrebs arzneimittelresistent ist; oder

der Krebs Lungenkrebs ist und die pharmazeutische Kombination den PARP-Inhibitor Olaparib umfasst; oder

der Krebs Bauchspeicheldrüsenkrebs ist und die pharmazeutische Kombination den PARP-Inhibitor Olaparib umfasst; oder

der Krebs Lungenkrebs ist und die pharmazeutische Kombination den PARP-Inhibitor Talazoparib umfasst; oder

der Krebs Bauchspeicheldrüsenkrebs ist und die pharmazeutische Kombination den PARP-Inhibitor Talazoparib umfasst.

8. Pharmazeutische Kombination zur Verwendung nach einem der vorhergehenden Ansprüche, wobei das Verhältnis des DNA-Ligase-IV-Inhibitors zu dem PARP-Inhibitor aus einem beliebigen von 1000:1 bis 1:1000, 500:1 bis 1:500, 100:1 bis 1:100, 50:1 bis 1:50, 20:1 bis 1:20, 10:1 bis 1:10, 5:1 bis 1:5, 2:1 bis 1:2, 1:1,5 bis 1,5:1 und 1:1 ausgewählt ist.

9. Pharmazeutische Kombination zur Verwendung nach einem der vorhergehenden Ansprüche, wobei der DNA-Ligase IV-Inhibitor in einer größeren molaren Menge vorliegt als der PARP-Inhibitor.

10. Pharmazeutische Kombination zur Verwendung nach Anspruch 7, wobei das Verhältnis des DNA-Ligase-IV-Inhibitors zu dem PARP-Inhibitor aus einem beliebigen von 1000:1 bis 1:1000, 500:1 bis 1:500, 100:1 bis 1:100, 50:1 bis 1:50, 20:1 bis 1:20, 10:1 bis 1:10, 5:1 bis 1:5, 2:1 bis 1:2, 1:1,5 bis 1,5:1 und 1:1 ausgewählt ist.

11. Pharmazeutische Kombination zur Verwendung nach Anspruch 7 oder 8, wobei der DNA-Ligase-IV-Inhibitor in einer größeren molaren Menge vorliegt als der PARP-Inhibitor.

12. Pharmazeutische Kombination zur Verwendung bei der Behandlung von Krebs nach einem der vorhergehenden Ansprüche, wobei die Kombination zur oralen Verabreichung oder parenteralen (z. B. intravenösen) Verabreichung formuliert ist.

## Revendications

1. Combinaison pharmaceutique destinée à être utilisée dans le traitement du cancer comprenant un inhibiteur de l'ADN ligase IV et un inhibiteur de PARP en quantités synergiquement efficaces pour une administration simultanée, séparée ou séquentielle à un sujet en ayant besoin, dans laquelle le cancer est un cancer résistant aux médicaments, dans laquelle l'inhibiteur de l'ADN ligase IV comprend le L189 ou le SCR7 et l'inhibiteur de PARP comprend le Talazoparib ou l'Olaparib.

2. Combinaison pharmaceutique destinée à être utilisée selon la revendication 1, dans laquelle l'inhibiteur de l'ADN ligase IV comprend un inhibiteur de l'ADN ligase IV humaine.

3. Combinaison pharmaceutique destinée à être utilisée selon la revendication 1 ou 2, dans laquelle

l'inhibiteur de l'ADN ligase IV comprend le L189 et l'inhibiteur de PARP comprend l'Olaparib ; ou
l'inhibiteur de l'ADN ligase IV comprend le L189 et l'inhibiteur de PARP comprend le Talazoparib ; ou
l'inhibiteur de l'ADN ligase IV comprend le SCR7 et l'inhibiteur de PARP comprend l'Olaparib ; ou
l'inhibiteur de l'ADN ligase IV comprend le SCR7 et l'inhibiteur PARP comprend le Talazoparib.

4. Combinaison pharmaceutique destinée à être utilisée dans le traitement du cancer selon l'une quelconque des revendications précédentes, dans laquelle le cancer est choisi parmi le cancer du poumon, cancer du sein, cancer du pancréas, cancer de l'utérus, cancer des ovaires, cancer de la peau (par exemple le cancer du mélanome) ou cancer colorectal.

5. Combinaison pharmaceutique destinée à être utilisée dans le traitement du cancer du poumon selon la revendication 4, dans laquelle :

le cancer du poumon est choisi parmi le carcinome pulmonaire à petites cellules, carcinome combiné à petites cellules, adénocarcinome, carcinome épidermoïde, carcinome à grandes cellules, cancer du poumon non à petites cellules indifférencié, mésothéliome ou tumeurs neuroendocrines ; ou
le cancer du sein est choisi parmi le cancer du sein métastatique, carcinome canalaire in situ (CCIS), carcinome lobulaire in situ (CLIS), cancer du sein canalaire invasif, cancer du sein lobulaire invasif, cancer du sein inflammatoire, cancer du sein triple négatif, carcinome médullaire, carcinome tubulaire, carcinome mucineux, tumeur maligne Phyllodes ou maladie de Paget ; ou
le cancer du pancréas est choisi parmi l'adénocarcinome canalaire pancréatique (PDAC), carcinome épidermoïde, carcinome adénosquameux, carcinome à cellules acineuses, néoplasme pseudopapillaire solide, pancréatoblastome, lésions précancéreuses bénignes ou tumeurs neuroendocrines pancréatiques ; ou
le cancer de la peau est choisi parmi le carcinome basocellulaire, carcinome épidermoïde ou mélanome ; ou
le cancer de l'ovaire est choisi parmi le cancer épithélial de l'ovaire, cancer stromal de l'ovaire et cancer de l'ovaire à cellules germinales.

6. Combinaison pharmaceutique destinée à être utilisée dans le traitement du cancer selon l'une quelconque des revendications 1 à 5, dans laquelle le cancer résistant aux médicaments est choisi parmi le cancer du sein, du poumon ou du pancréas.

7. Combinaison pharmaceutique destinée à être utilisée dans le traitement du cancer selon l'une quelconque des revendications 1 à 3, dans laquelle

le cancer est un cancer du poumon et la combinaison pharmaceutique comprend l'inhibiteur d'ADN ligase L189 et l'inhibiteur de PARP Olaparib, et dans laquelle le cancer du poumon est résistant aux médicaments ; ou
dans laquelle le cancer est un cancer du poumon et la combinaison pharmaceutique comprend l'inhibiteur d'ADN ligase L189 et l'inhibiteur de PARP Talazoparib, et dans laquelle le cancer du poumon est résistant aux médicaments ; ou
le cancer est un cancer du sein et la combinaison pharmaceutique comprend l'inhibiteur d'ADN ligase SCR7 et l'inhibiteur de PARP Olaparib, et dans laquelle le cancer du poumon est résistant aux médicaments ; ou
le cancer est un cancer du poumon et la combinaison pharmaceutique comprend l'inhibiteur de PARP Olaparib ; ou
le cancer est un cancer du pancréas et la combinaison pharmaceutique comprend l'inhibiteur de PARP Olaparib ; ou
le cancer est un cancer du poumon et la combinaison pharmaceutique comprend l'inhibiteur de PARP Talazoparib ; ou
le cancer est un cancer du pancréas et la combinaison pharmaceutique comprend l'inhibiteur de PARP Talazoparib.

8. Combinaison pharmaceutique destinée à être utilisée selon l'une quelconque des revendications précédentes, dans laquelle le rapport de l'inhibiteur de l'ADN ligase IV à l'inhibiteur de PARP est choisi parmi l'un quelconque de 1000:1 à 1:1000, 500:1 à 1:500, 100:1 à 1:100, 50:1 à 1:50, 20:1 à 1:20, 10:1 à 1:10, 5:1 à 1:5, 2:1 à 1:2, 1:1,5 à 1,5:1 et 1:1.

9. Combinaison pharmaceutique destinée à être utilisée selon l'une quelconque des revendications précédentes, dans laquelle l'inhibiteur de l'ADN ligase IV est présent en une quantité molaire supérieure à celle de l'inhibiteur de PARP.

10. Combinaison pharmaceutique destinée à être utilisée selon la revendication 7, dans laquelle le rapport de l'inhibiteur de l'ADN ligase IV à l'inhibiteur de PARP est choisi parmi l'un quelconque de 1000:1 à 1:1000, 500:1 à 1:500, 100:1 à 1:100, 50:1 à 1:50, 20:1 à 1:20, 10:1 à 1:10, 5:1 à 1:5, 2:1 à 1:2, 1:1,5 à 1,5:1 et 1:1.

11. Combinaison pharmaceutique destinée à être utilisée selon la revendication 7 ou 8, dans laquelle l'inhibiteur de l'ADN ligase IV est présent en une quantité molaire supérieure à celle de l'inhibiteur de PARP.

12. Combinaison pharmaceutique destinée à être utilisée dans le traitement du cancer selon l'une quelconque des revendications précédentes, dans laquelle la combinaison est formulée pour une administration orale ou parentérale (par exemple intraveineuse).

PLATE 1

| | 1 | 2 | 3 | 4 | 5 | 6 | 7 | 8 | 9 | 10 | 11 | 12 |
|---|---|---|---|---|---|---|---|---|---|---|---|---|
| A | OLA 20 µM SCR7 60 µM | OLA 20 µM SCR7 60 µM | OLA 20 µM SCR7 60 µM | OLA 20 µM SCR7 60 µM | OLA 20 µM SCR7 60 µM | OLA 20 µM SCR7 60 µM | OLA 20 µM L189 60 µM | OLA 20 µM L189 20 µM | OLA 20 µM L189 6.67 µM | OLA 20 µM L189 2.22 µM | OLA 20 µM L189 0.74 µM | OLA 20 µM L189 0.25 µM |
| B | OLA 6.67 µM SCR7 60 µM | OLA 6.67 µM SCR7 20 µM | OLA 6.67 µM SCR7 6.67 µM | OLA 6.67 µM SCR7 2.22 µM | OLA 6.67 µM SCR7 0.74 µM | OLA 6.67 µM SCR7 0.25 µM | OLA 6.67 µM L189 60 µM | OLA 6.67 µM L189 20 µM | OLA 6.67 µM L189 6.67 µM | OLA 6.67 µM L189 2.22 µM | OLA 6.67 µM L189 0.74 µM | OLA 6.67 µM L189 0.25 µM |
| C | OLA 2.22 µM SCR7 60 µM | OLA 2.22 µM SCR7 20 µM | OLA 2.22 µM SCR7 6.67 µM | OLA 2.22 µM SCR7 2.22 µM | OLA 2.22 µM SCR7 0.74 µM | OLA 2.22 µM SCR7 0.25 µM | OLA 2.22 µM L189 60 µM | OLA 2.22 µM L189 20 µM | OLA 2.22 µM L189 6.67 µM | OLA 2.22 µM L189 2.22 µM | OLA 2.22 µM L189 0.74 µM | OLA 2.22 µM L189 0.25 µM |
| D | OLA 0.74 µM SCR7 60 µM | OLA 0.74 µM SCR7 20 µM | OLA 0.74 µM SCR7 6.67 µM | OLA 0.74 µM SCR7 2.22 µM | OLA 0.74 µM SCR7 0.74 µM | OLA 0.74 µM SCR7 0.25 µM | OLA 0.74 µM L189 60 µM | OLA 0.74 µM L189 20 µM | OLA 0.74 µM L189 6.67 µM | OLA 0.74 µM L189 2.22 µM | OLA 0.74 µM L189 0.74 µM | OLA 0.74 µM L189 0.25 µM |
| E | OLA 0.25 µM SCR7 60 µM | OLA 0.25 µM SCR7 20 µM | OLA 0.25 µM SCR7 6.67 µM | OLA 0.25 µM SCR7 2.22 µM | OLA 0.25 µM SCR7 0.74 µM | OLA 0.25 µM SCR7 0.25 µM | OLA 0.25 µM L189 60 µM | OLA 0.25 µM L189 20 µM | OLA 0.25 µM L189 6.67 µM | OLA 0.25 µM L189 2.22 µM | OLA 0.25 µM L189 0.74 µM | OLA 0.25 µM L189 0.25 µM |
| F | OLA 0.08 µM SCR7 60 µM | OLA 0.08 µM SCR7 20 µM | OLA 0.08 µM SCR7 6.67 µM | OLA 0.08 µM SCR7 2.22 µM | OLA 0.08 µM SCR7 0.74 µM | OLA 0.08 µM SCR7 0.25 µM | OLA 0.08 µM L189 60 µM | OLA 0.08 µM L189 20 µM | OLA 0.08 µM L189 6.67 µM | OLA 0.08 µM L189 2.22 µM | OLA 0.08 µM L189 0.74 µM | OLA 0.08 µM L189 0.25 µM |
| G | OLA 20 µM | OLA 20 µM | OLA 6.67 µM | OLA 2.22 µM | OLA 0.74 µM | OLA 0.25 µM | TALA 60 µM | TALA 20 µM | TALA 6.67 µM | TALA 2.22 µM | TALA 0.74 µM | TALA 0.25 µM |
| H | DMSO 0.1% | DMSO 0.1% | DMSO 0.1% | DMSO 0.1% | CELL | CELL | CELL | CELL | BLANK | BLANK | BLANK | BLANK |

Fig. 1

EP 3 897 605 B1

PLATE 2

| | 1 | 2 | 3 | 4 | 5 | 6 | 7 | 8 | 9 | 10 | 11 | 12 |
|---|---|---|---|---|---|---|---|---|---|---|---|---|
| A | TALA 20 µM SCR7 60 µM | TALA 20 µM SCR7 20 µM | TALA 20 µM SCR7 6.67 µM | TALA 20 µM SCR7 2.22 µM | TALA 20 µM SCR7 0.74 µM | TALA 20 µM SCR7 0.25 µM | TALA 20 µM L189 60 µM | TALA 20 µM L189 20 µM | TALA 20 µM L189 6.67 µM | TALA 20 µM L189 2.22 µM | TALA 20 µM L189 0.74 µM | TALA 20 µM L189 0.25 µM |
| B | TALA 6.67 µM SCR7 60 µM | TALA 6.67 µM SCR7 20 µM | TALA 6.67 µM SCR7 6.67 µM | TALA 6.67 µM SCR7 2.22 µM | TALA 6.67 µM SCR7 0.74 µM | TALA 6.67 µM SCR7 0.25 µM | TALA 6.67 µM L189 60 µM | TALA 6.67 µM L189 20 µM | TALA 6.67 µM L189 6.67 µM | TALA 6.67 µM L189 2.22 µM | TALA 6.67 µM L189 0.74 µM | TALA 6.67 µM L189 0.25 µM |
| C | TALA 2.22 µM SCR7 60 µM | TALA 2.22 µM SCR7 20 µM | TALA 2.22 µM SCR7 6.67 µM | TALA 2.22 µM SCR7 2.22 µM | TALA 2.22 µM SCR7 0.74 µM | TALA 2.22 µM SCR7 0.25 µM | TALA 2.22 µM L189 60 µM | TALA 2.22 µM L189 20 µM | TALA 2.22 µM L189 6.67 µM | TALA 2.22 µM L189 2.22 µM | TALA 2.22 µM L189 0.74 µM | TALA 2.22 µM L189 0.25 µM |
| D | TALA 0.74 µM SCR7 60 µM | TALA 0.74 µM SCR7 20 µM | TALA 0.74 µM SCR7 6.67 µM | TALA 0.74 µM SCR7 2.22 µM | TALA 0.74 µM SCR7 0.74 µM | TALA 0.74 µM SCR7 0.25 µM | TALA 0.74 µM L189 60 µM | TALA 0.74 µM L189 20 µM | TALA 0.74 µM L189 6.67 µM | TALA 0.74 µM L189 2.22 µM | TALA 0.74 µM L189 0.74 µM | TALA 0.74 µM L189 0.25 µM |
| E | TALA 0.25 µM SCR7 60 µM | TALA 0.25 µM SCR7 20 µM | TALA 0.25 µM SCR7 6.67 µM | TALA 0.25 µM SCR7 2.22 µM | TALA 0.25 µM SCR7 0.74 µM | TALA 0.25 µM SCR7 0.25 µM | TALA 0.25 µM L189 60 µM | TALA 0.25 µM L189 20 µM | TALA 0.25 µM L189 6.67 µM | TALA 0.25 µM L189 2.22 µM | TALA 0.25 µM L189 0.74 µM | TALA 0.25 µM L189 0.25 µM |
| F | TALA 0.08 µM SCR7 60 µM | TALA 0.08 µM SCR7 20 µM | TALA 0.08 µM SCR7 6.67 µM | TALA 0.08 µM SCR7 2.22 µM | TALA 0.08 µM SCR7 0.74 µM | TALA 0.08 µM SCR7 0.25 µM | TALA 0.08 µM L189 60 µM | TALA 0.08 µM L189 20 µM | TALA 0.08 µM L189 6.67 µM | TALA 0.08 µM L189 2.22 µM | TALA 0.08 µM L189 0.74 µM | TALA 0.08 µM L189 0.25 µM |
| G | SCR7 60 µM | SCR7 20 µM | SCR7 6.67 µM | SCR7 2.22 µM | SCR7 0.74 µM | SCR7 0.25 µM | L189 60 µM | L189 20 µM | L189 6.67 µM | L189 2.22 µM | L189 0.74 µM | L189 0.25 µM |
| H | DMSO 0.1% | DMSO 0.1% | DMSO 0.1% | DMSO 0.1% | CELL | CELL | CELL | CELL | BLANK | BLANK | BLANK | BLANK |

# Fig. 1 (continued)

Raw data:

| A549-2/1 | 1 | 2 | 3 | 4 | 5 | 6 | 7 | 8 | 9 | 10 | 11 | 12 |
|---|---|---|---|---|---|---|---|---|---|---|---|---|
| A | 1546531 | 2927285 | 3208782 | 3425188 | 3621343 | 3919394 | 1569804 | 2912912 | 3693344 | 3874593 | 4273306 | 4687036 |
| B | 2099151 | 3612964 | 4102705 | 4065456 | 4377794 | 4575500 | 1780154 | 2968181 | 3630872 | 3916730 | 4449009 | 4651599 |
| C | 2003684 | 4180327 | 4799944 | 4526796 | 4525944 | 4781793 | 2013120 | 3141409 | 3498840 | 3893980 | 3970283 | 5066957 |
| D | 1649555 | 3423998 | 4251873 | 4238455 | 4026602 | 4357733 | 1801000 | 2802764 | 3996794 | 3771262 | 3831225 | 4586500 |
| E | 1658748 | 3093695 | 3893784 | 4063987 | 4293179 | 4343803 | 1628895 | 2792921 | 3553254 | 3760402 | 3783571 | 4650330 |
| F | 1412123 | 3242792 | 3841462 | 4053395 | 4167662 | 4523404 | 1667541 | 2896346 | 3709232 | 4017299 | 4050038 | 4351850 |
| G | 1704089 | 3256449 | 4190838 | 4146142 | 4296500 | 4456620 | 1086451 | 1180976 | 1354432 | 1606895 | 2159110 | 3114319 |
| H | 4566339 | 4512294 | 4778291 | 4499396 | 4578846 | 4722216 | 4329931 | 4393820 | 2976 | 1289 | 1519 | 1390 |

# Fig. 2

Blank corrected:

| A549-2/1 | 1 | 2 | 3 | 4 | 5 | 6 | 7 | 8 | 9 | 10 | 11 | 12 |
|---|---|---|---|---|---|---|---|---|---|---|---|---|
| A | 1544239 | 2924993 | 3206490 | 3422896 | 3619051 | 3917102 | 1567512 | 2910620 | 3691052 | 3872301 | 4271014 | 4684744 |
| B | 2096859 | 3610672 | 4100413 | 4063164 | 4375502 | 4573208 | 1777862 | 2965889 | 3628580 | 3914438 | 4446717 | 4649307 |
| C | 2001392 | 4178035 | 4797652 | 4524504 | 4523652 | 4779501 | 2010828 | 3139117 | 3496548 | 3891688 | 3967991 | 5064665 |
| D | 1647263 | 3421706 | 4249581 | 4236163 | 4024310 | 4355441 | 1798708 | 2800472 | 3994502 | 3768970 | 3828933 | 4584208 |
| E | 1656456 | 3091403 | 3891492 | 4061695 | 4290887 | 4341511 | 1626603 | 2790629 | 3550962 | 3758110 | 3781279 | 4648038 |
| F | 1409831 | 3240500 | 3839170 | 4051103 | 4165370 | 4521112 | 1084159 | 2894054 | 3706940 | 4015007 | 4047746 | 4349558 |
| G | 1701797 | 3254157 | 4188546 | 4143850 | 4294208 | 4454328 | 1084159 | 1178684 | 1352140 | 1604603 | 2156818 | 3112027 |
| H | 4564047 | 4510002 | 4775999 | 4497104 | 4576554 | 4719924 | 4327639 | 4391528 | 684 | -1003 | -773 | -902 |

# Fig. 3

Treated/control % (T/C%):

| A549-2/1 | 1 | 2 | 3 | 4 | 5 | 6 | 7 | 8 | 9 | 10 | 11 | 12 |
|---|---|---|---|---|---|---|---|---|---|---|---|---|
| A | 34.18% | 64.73% | 70.96% | 75.75% | 80.09% | 86.69% | 34.69% | 64.42% | 81.69% | 85.70% | 94.52% | 103.68% |
| B | 46.41% | 79.91% | 90.75% | 89.92% | 96.84% | 101.21% | 39.35% | 65.64% | 80.31% | 86.63% | 98.41% | 102.90% |
| C | 44.29% | 92.47% | 106.18% | 100.13% | 100.11% | 105.78% | 44.50% | 69.47% | 77.38% | 86.13% | 87.82% | 112.09% |
| D | 36.46% | 75.73% | 94.05% | 93.75% | 89.06% | 96.39% | 39.81% | 61.98% | 88.40% | 83.41% | 84.74% | 101.45% |
| E | 36.66% | 68.42% | 86.12% | 89.89% | 94.96% | 96.08% | 36.00% | 61.76% | 78.59% | 83.17% | 83.68% | 102.87% |
| F | 31.20% | 71.72% | 84.97% | 89.66% | 92.19% | 100.06% | 36.85% | 64.05% | 82.04% | 88.86% | 89.58% | 96.26% |
| G | 37.66% | 72.02% | 92.70% | 91.71% | 95.04% | 98.58% | 23.99% | 26.09% | 29.92% | 35.51% | 47.73% | 68.87% |
| H | 101.01% | 99.81% | 105.70% | 99.53% | 101.29% | 104.46% | 95.78% | 97.19% | 0.02% | -0.02% | -0.02% | -0.02% |

# Fig. 4

Olaparib vs. Olaparib + 60uM SCR7 vs. BLISS theoretical combination kill-rate (BT474)

**Fig. 5**

Talazoparib vs. Talazoparib + 60uM L189 vs. BLISS theoretical combination kill-rate (H1975)

**Fig. 6**

Olaparib vs. Olaparib + 60uM L189 vs. BLISS theoretical combination kill-rate (H1975)

**Fig. 7**

**siRNA - 8 days**

Fig. 8

Fig. 9

Fig. 10

Fig. 11

Fig. 12

Fig. 13

Fig. 14

| | 1 | 2 | 3 | 4 | 5 | 6 | 7 | 8 | 9 | 10 | 11 | 12 | | | | |
|---|---|---|---|---|---|---|---|---|---|---|---|---|---|---|---|---|
| A | 0 | 60 | 20 | 6.67 | 2.22 | 0.741 | 0.247 | 0.082 | 0.027 | 0.009 | 0.003 | 0 | OLA (uM) | | DNA ligase 4 siRNA | |
| B | 0 | 60 | 20 | 6.77 | 2.22 | 0.741 | 0.247 | 0.082 | 0.027 | 0.009 | 0.003 | 0 | | | | |
| C | 0 | 60 | 20 | 6.67 | 2.22 | 0.741 | 0.247 | 0.082 | 0.027 | 0.009 | 0.003 | 0 | TALA (uM) | | | |
| D | 0 | 60 | 20 | 6.67 | 2.22 | 0.741 | 0.247 | 0.082 | 0.027 | 0.009 | 0.003 | 0 | | | | |
| E | 0 | 60 | 20 | 6.67 | 2.22 | 0.741 | 0.247 | 0.082 | 0.027 | 0.009 | 0.003 | 0 | OLA (uM) | | non-targeting siRNA | |
| F | 0 | 60 | 20 | 6.67 | 2.22 | 0.741 | 0.247 | 0.082 | 0.027 | 0.009 | 0.003 | 0 | | | | |
| G | 0 | 60 | 20 | 6.67 | 2.22 | 0.741 | 0.247 | 0.082 | 0.027 | 0.009 | 0.003 | 0 | TALA (uM) | | | |
| H | 0 | 60 | 20 | 6.67 | 2.22 | 0.741 | 0.247 | 0.082 | 0.027 | 0.009 | 0.003 | 0 | | | | |

Fig. 15

UACC-62 with 1000 cells/well (UA1679), S3766-1-X x Olaparib

Fig. 16

Fig. 17

RED_tris-NTA labeled LIG4 (DVU1-DVV1, PC13347-1) vs. TAI-04 (S3766-1-X)

| Fluorophore | Fluor. Molecule | Titrant | $K_D$ [M] | Lower confidence [M] | Lower confidence [M] | ΔFnorm [%] | Signal / Noise | TRIC On [s] |
|---|---|---|---|---|---|---|---|---|
| RED-tris-NTA | PC13347-1 | S3766-1-X | 7.2E-06 | 3.7E-06 | 1.39E-05 | 10.1 | 9.9 | 5 |

RED_tris-NTA labeled LIG4 bind S3766-1 with a determined $K_D$ of 7.2 µM.

Fig. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008124838 A
- WO 2018067575 A
- US 9132120 B

**Non-patent literature cited in the description**

- **CZYZ MALGORZATA et al.** *Oncotarget,* 2016, vol. 7, 75551-75560
- **PASCAL JM ; O'BRIEN PJ ; TOMKINSON AE ; ELLENBERGER T.** Human DNA ligase I completely encircles and partially unwinds nicked DNA. *Nature,* November 2004, vol. 432 (7016), 473-8
- **HA HC ; SNYDER SH.** Poly (ADP-ribose) polymerase-1 in the nervous system. *Neurobiology of Disease.,* August 2000, vol. 7 (4), 225-39